# EUROPEAN PATENT SPECIFICATION

(11) **EP 0 502 036 B1**
(45) Date of publication and mention of the grant of the patent: **20.12.1995**
(21) Application number: 90917236.3
(22) Date of filing: 30.10.1990
(51) Int. Cl.: C12N 15/16, C12N 15/12, C07K 14/495, A61K 38/18, G01N 33/74, C12N 1/21, C12N 5/10

(54) **LATENCY ASSOCIATED PEPTIDE AND USES THEREFOR**
LATENZ ASSOZIIERTE PEPTIDE UND DEREN VERWENDUNG
PEPTIDE ASSOCIE A LA LATENCE ET UTILISATIONS

(30) Priority: 22.11.1989 US 441680
(43) Date of publication of application: 09.09.1992
(73) Proprietor: GENENTECH, INC., South San Francisco California 94080 (US)
(72) Inventor: LEVINSON, Arthur, Hillsborough, CA 94010 (US); HAMMONDS, Glenn, R., Berkeley, CA 94705 (US); MASON, Anthony, Burlingame, CA 94010 (US)
(74) Representative: Armitage, Ian Michael
(86) International application number: US9006292
(87) International publication number: WO9108291

(56) References cited:
- EP-A- 0 269 408
- EP-A- 0 293 785
- EP-A- 0 356 935
- EP-A- 0 376 785
- FEBS Letters, vol. 242, no. 2, January 1989, Elsevier Science Publishers B.V. (Biomedical Div); F. OKADA et al., pp. 240-244
- SCIENCE, vol. 247, 16 March 1990; A.M. GRAY et al., pp. 1328-1330
- JOURNAL OF BIOLOGICAL CHEMISTRY, vol. 263, no. 16, 05 June 1988, US;L.M. WAKEFIELD et al., pp. 7646-7654

## Description

### Background of the Invention

### Field of the Invention

This invention relates to a latency associated peptide and to the means and methods for its production in therapeutically significant quantities, as well as to uses for the peptide.

### Description of the Background Art

The beta transforming growth factors (TGF-βs) are multifunctional cytokines, produced by many types of cells, including hematopoietic, neural, heart, fibroblast, and tumor cells, that can regulate the growth and differentiation of cells from a variety of tissue origins (Sporn *et al*., Science, 233: 532 [1986]) and stimulate the formation and elaboration of various stromal elements. Of particular interest from an immunological viewpoint are the potent immunosuppressive activities of TGF-β, which include lymphokine activated killer (LAK) and cytotoxic T lymphocyte (CTL) inhibition (Ranges *et al*., J. Exp. Med., 166: 991 [1987], Espevik *et al*., J. Immunol., 140: 2312 [1988], Grimm *et al*., Cancer Immunol. Immunother., 27: 53 [1988], Kasid *et al*., J. Immunol., 141: 690 [1988], Mule *et al*., Cancer Immunol. Immunother., 26: 95 [1988]), depressed B cell lymphopoiesis and kappa light chain expression (Lee *et al*., J. Exp. Med., 166: 1290 [1987]), negative regulation of hematopoiesis (Hino *et al*., Br. J. Haematol., 70: 143 [1988], Sing *et al*., Blood, 72: 1504 [1988]), down-regulation of HLA-DR expression on tumor cells (Czarniecki *et al*., J. Immunol., 140: 4217 [1988], Zuber *et al*., Eur. J. Immunol., 18: 1623 [1988]), and inhibition of the proliferation of antigen-activated B lymphocytes in response to B cell growth factor (Petit-Koskas *et al.,* Eur. J. Immunol., 18: 111 [1988]). The observation that many human tumors (deMartin *et al*., EMBO J., 6: 3673 [1987], Kuppner *et al*., Int. J. Cancer, 42: 562 [1988]) and many tumor cell lines (Derynck *et al*., Cancer Res., 47: 707 [1987], Roberts *et al.*, Br. J. Cancer, 57: 594 [1988]) produce TGF-β suggests a possible mechanism for those tumors to evade normal immunological surveillance. This negative immunomodulation, coupled with the observations that certain transformed cell lines have lost the ability to respond to TGF-β in an autocrine fashion (Wakefield *et al*., J. Cell Biol., 105: 965 [1987], McMahon *et al*., Cancer Res., 46: 4665 [1986]), and that TGF-β stimulates stroma formation, and decreases immune surveillance of the tumor, suggests attractive models for neoplasm deregulation and proliferation (Roberts *et al*., Br. J. Cancer, supra).

There are at least five forms of TGF-β currently identified, TGF-β1, TGF-β2, TGF-β3, TGF-β4, and TGF-β5. Suitable methods are known for purifying this family of TGF-βs from various species such as human, mouse, green monkey, pig, bovine, chick, and frog, and from various body sources such as bone, platelets, or placenta, for producing it in recombinant cell culture, and for determining its activity. See, for example, R. Derynck *et al*., Nature, 316:701-705 [1985]; European Pat. Pub. Nos. 200,341 published December 10, 1986, 169,016 published January 22, 1986, 268,561 published May 25, 1988, and 267,463 published May 18, 1988; U.S. Pat. No. 4,774,322; Cheifetz et al, Cell, 48: 409-415 [1987]; Jakowlew *et al*., Molecular Endocrin., 2: 747-755 [1988]; Dijke *et al*., Proc. Natl. Acad. Sci. (U.S.A.), 85: 4715-4719 [1988]; Derynck *et al*., J. Biol. Chem., 261: 4377-4379 [1986]; Sharples *et al*., DNA, 6: 239-244 [1987]; Derynck *et al*., Nucl. Acids. Res., 15: 3188-3189 [1987]; Derynck *et al*., Nucl. Acids. Res., 15: 3187 [1987]; Derynck *et al*., EMBO J., 7: 3737-3743 [1988]; Seyedin *et al*., J. Biol. Chem., 261: 5693-5695 [1986]; Madisen *et a*l., DNA, 7: 1-8 [1988]; and Hanks *et al*., Proc. Natl. Acad. Sci. (U.S.A.), 85: 79-82 [1988].

The activated form of TGF-β1 is a homodimer formed by dimerization of the carboxy-terminal 112 amino acids of a 390 amino acid precursor (Derynck *et al*., Nature, supra). TGF-β2 has a precursor form of 414 amino acids and is also processed to a homodimer from the carboxy-terminal 112 amino acids that shares approximately 70% homology with the active form of TGF-β1 (Marquardt *et al*., J. Biol. Chem., 262: 12127 [1987]). TGF-β2 has been purified from porcine platelets (Seyedin *et al*., J. Biol. Chem., 262: 1946-1949 [1987]) and human glioblastoma cells (Wrann *et al.*, EMBO J., 6: 1633 [1987]) and recombinant human TGF-β2 has been cloned (deMartin *et al*., supra). Recombinant TGF-β1 has been cloned (Derynck *et al*., Nature, supra) and expressed in Chinese hamster ovary cells (Gentry *et al*., Mol. Cell. Biol., 7: 3418-3427 [1987]).

TGF-β3, TGF-β4, and TGF-β5, which are the most recently discovered forms of TGF-β, were identified by screening cDNA libraries. None of these three putative proteins has been isolated from natural sources, although Northern blots demonstrate expression of the corresponding mRNAs. Human and porcine TGF-β3 have been cloned and described previously (Derynck *et al*., EMBO J.,7: 3737-3743 (1988), ten Dijke *et al*., Proc. Natl. Acad. Sci. USA, 85: 4715 [1988]). TGF-β4 and TGF-β5 were cloned from a chicken chondrocyte cDNA library (Jakowlew *et al*., Molec. Endocrinol., 2: 1186-1195 [1988]) and from a frog oocyte cDNA library, respectively. The frog oocyte cDNA library can be screened using a probe derived from one or more sequences of another type of TGF-β. TGF-β4 mRNA is detectable in chick embryo chondrocytes, but is far less abundant than TGF-β3 mRNA in developing embryos or in chick embryo fibroblasts. TGF-β5 mRNA is expressed in frog embryos beyond the neurula state and in Xenopus tadpole (XTC) cells.

TGF-β has been shown to have numerous regulatory actions on a wide variety of both normal and neoplastic cells. TGF-β is multifunctional, as it can either stimulate or inhibit cell proliferation, differentiation, and other critical processes in cell function (M. Sporn, Science, 233:532 [1986]). For a general review of TGF-β and its actions, see Sporn *et al.*, J. Cell Biol., 105: 1039-1045 (1987), Sporn and Roberts, Nature, 332: 217-219 (1988), and Roberts *et al.*, Recent Progress in Hormone Research, 44: 157-197 (1988).

Natural TGF-β1 is made predominantly, if not exclusively, in a biologically latent form, which can be activated *in vitro* by denaturants such as urea, heat, plasmin, high salt, endoglycosidase F, cathepsin D, type IV collagenase, cocultured endothelial cells and pericytes, plasminogen activators such as urokinase, stimulated osteoclasts, or extremes of pH. See, e.g., Pircher *et al.,* Canc. Res., 44: 5538-5543 (1984) re latent TGF-β from nontransformed and Kirsten sarcoma virus-transformed normal rat kidney cells; Antonelli-Orlidge *et al.*, Proc. Natl. Acad. Sci. USA, 86: 4544-4548 (1989) re latent TGF-β from pericytes and capillary endothelial cells; Lawrence *et al*., Biochem. Biophys. Res. Commun., 133: 1026-1034 (1985) re latent TGF-β from chicken embryo fibroblasts; Oreffo *et al.*, Biochem. Biophys. Res. Commun., 158: 817-823 (1989) re latent TGF-β from murine bone organ cultures; Keski-Oja *et al*., J. Cell Biol., 107: (6 Part 3), 1988, 50a re latent TGF-β from human lung adenocarcinoma cell line; Miyazono and Heldin, J. Cell. Biochem. Supp. 0 (13 part B) 1989, p. 92 and Miyazono and Heldin, Nature, 338: 158-160 (1989) re latent TGF-β from human platelets and its carbohydrate structure; and Pircher *et al*., Biochem. Biophys. Res. Commun., 136: 30-37 (1986) re latent TGF-β from human blood platelets. See also Lawrence *et al*., J. Cell. Physiol., 121: 184-188 (1984); Kryceve-Martinerie *et al*., Int. J. Cancer, 35: 553-558 (1985); Brown *et al*., "TGF-β", NY Acad. Sci. Meeting Abstract, May 18-20, 1989; Danielpour *et al*., J. Cell. Physiol., 138: 79-86 (1989); Wakefield *et al*., J. Biol. Chem., 263: 7646-7654 (1988); and Miyazono *et al*., J. Biol. Chem., 263: 6407-6415 (1988).

Several groups have characterized the latent form of TGF-β1 secreted by human platelets. Pircher *et al*., 1986, supra stated that it has an apparent molecular weight of 400 Kd. More recently, it has been characterized as a three-component complex of about 235 Kd, wherein the active TGF-β1 (25 Kd dimer) is non-covalently associated with the remainder of the processed precursor (75 Kd dimer), which in turn is disulfide-bonded to an unrelated protein of 125-160 Kd (Wakefield *et al*., J. Biol. Chem., 263, supra; Miyazono *et al*., supra; Miyazono *et al*., J. Cell Biochem. Supp., 0 (12 Part A), 1988, p. 200; Wakefield *et al*,, J. Cell. Biochem. Suppl., 11A: 0, 46 [1987]). The function of the binding protein of 125-160 Kd remains to be elucidated. Recent characterizations indicate that it contains at least 14 EGF-like repeats and six potential N-glycosylation sites and calcium binding domains (Kanzaki *et al*., "TGF-β", NY Acad. Sci. meeting abstract, May 18-20, 1989; Miyazono, "TGF-β", NY Acad. Sci. meeting abstract, May 18-20, 1989). Latent TGF-β secreted by many cells in culture has a similar structure (Wakefield *et al*., J. Biol. Chem., supra), and this is the form in which TGF-β1 is probably perceived initially by target cells *in vivo*. It has been suggested that the precursor remainder of TGF-β may have an important independent biological function based on conservation of sequences in the precursor region (Roberts *et al*., Recent Progress in Hormone Research, supra). Additionally, a mutation at position 33 of precursor TGF-β1 is reported to increase the yield of mature TGF-β1, and dimerization of the precursor "pro" region is suggested as necessary to confer latency (Brunner *et al*., J. Biol. Chem., 264: 13660-13664 [1989]).

A latent form of TGF-β purified from porcine platelets as a high molecular weight complex was analyzed. The mature TGF-β dimer is non-covalently linked to a component of 210 Kd on non-reducing SDS-PAGE. Upon reduction with dithiothreitol, this large component was resolved into bands of 40 Kd and 115-130 Kd. Kamerud *et al*., J. Cell Biol., 107: (6 Part 3), Jan. 29-Feb. 2, 1989, 49a.

Another platelet-derived molecule able to form latent TGF-β is that described by Jap. Kokai No. 63-196600 published August 15, 1988. This is a glycoprotein subunit of about 46 Kd that in association with four or more molecules binds reversibly to TGF-β on platelets, controlling TGF-β activity, is stable to heat and acid, and loses its TGF-β-inhibition activity when treated with dithiothreitol or trypsin. An additional platelet-derived latent TGF-β is disclosed by lap. Kokai No. 63-150300 published June 22, 1988. This glycoprotein has a molecular weight of about 44 Kd, reversibly links to TGF-β to inhibit TGF-β activity, is stable to heat and acid, and loses TGF-β inhibiting activity by treating with dithiothreitol or trypsin.

An additional masking protein for TGF-β from rat platelets having a molecular weight of about 440 Kd is described by Nakamura *et al*., Biochem. Biophys. Res. Commun., 141: 176-184 (1986). Further analysis revealed the masking protein as consisting of two subunits of 39 Kd and 105-120 Kd linked by disulfide bonds to form a 180-210 Kd complex. Okada *et al*., Cell Struct. Funct., 13 (6): 615 (1988); Okada *et al*., FEBS Letters, 242: 240-244 (1989). The N-terminal sequence of the 39 Kd subunit was found to be identical to the N-terminal part of the TGF-β precursor starting 30 residues from the initiation site of the transcribed precursor. The 180-210 Kd component was found to have a masking activity, but the 39 Kd and 105-120 Kd components had no activity, leading the authors to conclude on p. 243 that the 180-210 Kd component is the minimum active unit for masking TGF-β.

The mechanism of activation *in vivo* is not known, but may involve proteolysis or dissociation (Lyons *et al*., J. Cell. Biol., 106: 1659-1665 (1988); Keski-Oja *et al*., J. Cell Biochem. Suppl., 11A: 0, 60 [1987]). Since the TGF-β1 receptor is almost ubiquitously expressed, target restriction for TGF-β1 action may be defined by the presence or absence of the activating mechanism rather than the receptor (Wakefield *et al*., J. Cell. Biol., 105: 965-975 [1987]), and it is anticipated that activation of the latent form will be a major regulatory step in TGF-β1 action.

Recently, it has been reported that the latent form of TGF-β in serum consists of TGF-β bound to α2-macroglobulin, which is a major serum protein involved in clearing serum endoproteases, including thrombin, nucleophiles, and trypsin, from the circulation. O'Connor and Wakefield, J. Biol. Chem., 262: 14090-14099 (1987); Huang *et al*., Fed. Proc., 46: (6) 2024 (1987); Huang *et al*., J. Biol. Chem., 263: 1535-1541 (1988). A differential inhibition of TGF-β1 and TGF-β2 activity by α2-macroglobulin was observed by Danielpour and Sporn, J. Cell Biochem., 13B: 83 (1989). It has been postulated that TGF-β1 is stored in platelets in a latent complex containing the NH2-terminal part of its precursor and TGF-β1 binding protein, and that other proteins, including α2-macroglobulin, can act as scavengers and inactivate released TGF-β1. Most recent findings indicate that the interaction of TGF-β with α2-macroglobulin does not led to the clearance of the TGF-β-α2-macroglobulin complex, as was previously postulated. Philip and O'Connor-McCourt, "TGF-β", NY Acad. Sci. meeting abstract, May 18-20, 1989. The authors suggest that the complex may represent a circulating latent form of TGF-β that plays an important role in the endocrine actions of TGF-β.

The entire TGF-β1 precursor coding region has been successfully expressed in Chinese hamster ovary cells (Gentry *et al*., supra, and Lyons *et al*., J. Cell Biol., 107 (6 part 3) 1988, 51a for simian TGF-β1) and in a human renal carcinoma cell line (Wakefield *et al*., J. Cell Biochem. Supp. 0 (13 Part B) Jan. 21-27, 1989, p. 100 and Wakefield *et al.*, Growth Factors, 1: (1989) for human TGF-β1). Like platelet TGF-β1, the recombinant TGF-β1 is secreted in a biologically latent form. The recombinant latent form requires transient acidification for activation. Immunoblot analysis and gel filtration indicate that the recombinant latent TGF-β1 is a 100 Kd complex in which active 25 Kd TGF-β is non-covalently associated with the remaining 75 Kd of the processed precursor from which it has been biosynthetically cleaved. Unlike the platelet latent complex, the recombinant latent complex contains no 135 Kd component. Gentry *et al*. detected high levels of precursor sequences lacking mature TGF-β in the conditioned medium of the transfected CHO cells that appeared to be quite stable.

Wakefield *et al*., Growth Factors, supra, observed the secretion in roughly equivalent proportions of another 100 Kd species of latent human TGF-β1, in which the 75 Kd and 25 Kd components are covalently linked by a disulfide bond. This molecular species appears to have little intrinsic bioactivity, although it does slowly release active TGF-β1 with time, probably through a disulfide exchange reaction. A similar disulfide-bonded species has been observed in other expression systems for TGF-β1 (Gentry *et al*., Mol. and Cell. Biol., 8: 4162-4168 [1988]), and it is not clear whether it is an artifact of the expression system, or a normal biosynthetic intermediate that accumulates due to an overloading of the cellular processing machinery.

The latent form of TGF-β2 has also recently been produced in COS cells transfected with the appropriate vector. Madisen *et al*., DNA, 8: 205-212 (1989). The authors found additionally that DNA encoding the latency associated peptide of TGF-β1 fused to DNA encoding mature TGF-β2 and expressed in CHO cells resulted in secreted mature TGF-β2 upon activation. Thus, the processing signals contained within the TGF-β1 amino-terminal precursor portion can function in producing mature TGF-β2.

Certain authors have stated that both platelets and cells in culture release TGF-β in a latent form that is irreversibly activated by acid treatment (Lyons *et al*., J. Cell Biochem. Suppl., 0 (12 part A), Jan. 24-30, 1988). Others have suggested, in the context of recombinant as well as natural latent TGF-β, that since activation is irreversible, there is probably a conformational charge of the precursor portion upon dissociation of the complex such that the 25 Kd active molecule can no longer bind to the precursor portion. (Lyons *et al.,* J. Cell Biol., 106, supra). Still other authors have characterized the activation of recombinant and platelet complexes as reversible (Wakefield *et al*., J. Cell Biochem. (1989), supra, and Wakefield *et al*., Growth Factors, supra).

In view of the pronounced inhibitory effect of the recombinant latency associated peptide of TGF-β on the actions of both exogenously administered and endogenously produced active TGF-β, it is an object of the present invention to provide such a peptide in commercially useful quantities from a therapeutically acceptable source for use in inhibiting *in vivo* the deleterious effects of active TGF-β, i.e., a latency associated peptide completely free of other naturally occurring (source) proteins.

It is an additional object to prepare amino acid sequence and other variants of the latency associated peptide that do not substantially adversely affect the biological activity of the peptide.

It is yet another object to produce a form of latent TGF-β by combining the latency associated peptide with mature TGF-β for therapeutic administration.

These and other objects of the invention will be apparent from the specification as a whole.

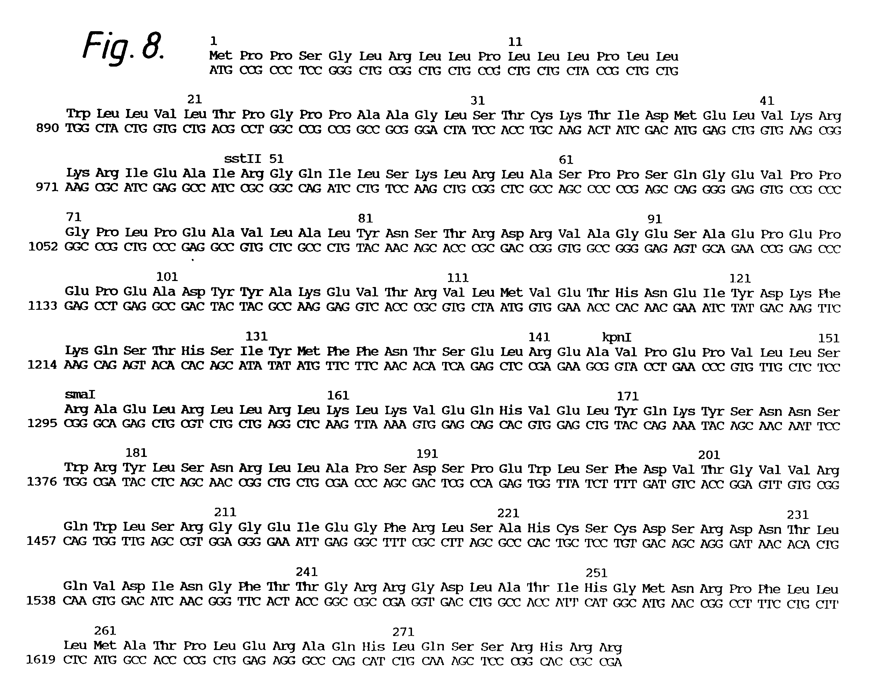

In one aspect, this invention provides an isolated nucleic acid sequence comprising a DNA sequence encoding the peptide shown in figure 8, or analogues or variants thereof, having the biological activity of a latency associated peptide, provided that the sequence does not also encode a mature TGF-β.

This invention also provides an expression vector comprising the above nucleic acid sequence operably linked to control sequences recognized by a host transformed by the vector and host cells transformed with this expression vector.

In another aspect, this invention provides a method of producing a latency associated peptide, which method comprises culturing the transformed host cells to express nucleic acid encoding the peptide in the host cell culture.

In a still further aspect, the invention provides latency associated peptide that is completely free of source proteins.

In addition, the invention provides a latency associated peptide for use in a method of medical treatment or diagnosis.

In a still further aspect the invention furnishes a pharmaceutical composition useful for antagonizing a TGF-β activity comprising a therapeutically effective amount of the above peptide in a pharmaceutically acceptable carrier.

In yet another aspect, the invention provides the use of a latency associated peptide in the preparation of a medicament for antagonizing a TGF-β activity in a mammal.

In a still further aspect the invention provides a pharmaceutical composition useful as having a TGF-β activity comprising a therapeutically effective amount of the above peptide and a therapeutically effective amount of a mature TGF-β in a pharmaceutically acceptable carrier.

In addition, the invention provides the use of a latency associated peptide and a TGF-β in the preparation of a medicament for treating a mammal in need of TGF-β treatment.

In yet another aspect, a method is provided for diagnosing the presence of a disease or disorder detectable by the pretence of mature TGF-β in serum sample of a patient comprising adding to the serum sample a labeled form of the latency associated peptide herein and assaying for the presence of labeled complex of the peptide and mature TGF-β in the serum sample.

The pretent invention renders possible the production of a heat-labile latency associated peptide and/or derivatives thereof by recombinant techniques, as well as products and method related to such production. It is believed that the latency associated peptide prepared by the method described herein is useful for controlling TGF-β activity, as by avenging and inactivating excess active TGF-β in the circulation, tissue, or elsewhere, whether from a exogenous or endogenous source. It can be used, for example, in situations where TGF-β activation has been implicated in the pathogenesis of certain diseases, such a that characterized by excessive fibrosis. In addition, the latency associated peptide is useful in treating other deleterious conditions caused by TGF-β or resulting from the production of TGF-β, such as in the suppression of tumors that grow autonomously by a autocrine mechanism involving TGF-β or in the reversal of immunosuppression. The peptide can also be used a a diagnostic agent.

Latent TGF-β can be made recombinantly by individually expressing and purifying the latency associated peptide and the active (mature) portion of TGF-β. When added together, these proteins reassociate to form an inactive (latent) complex. This complex may be useful in delivering TGF-β to sites capable of activating it, so that a different spectrum of activities will be seen with the latent form than that seen with mature TGF-β alone. In one such embodiment the complex is administered systemically to target TGF-β to its receptor sites where activation usually occurs, thus precluding unwanted system effusing and obtaining desirable TGF-β activities such as immunosuppression and bone formation.

Other uses for the latency associated peptide will be apparent to those skilled in the art.

### Brief Description of the Drawings

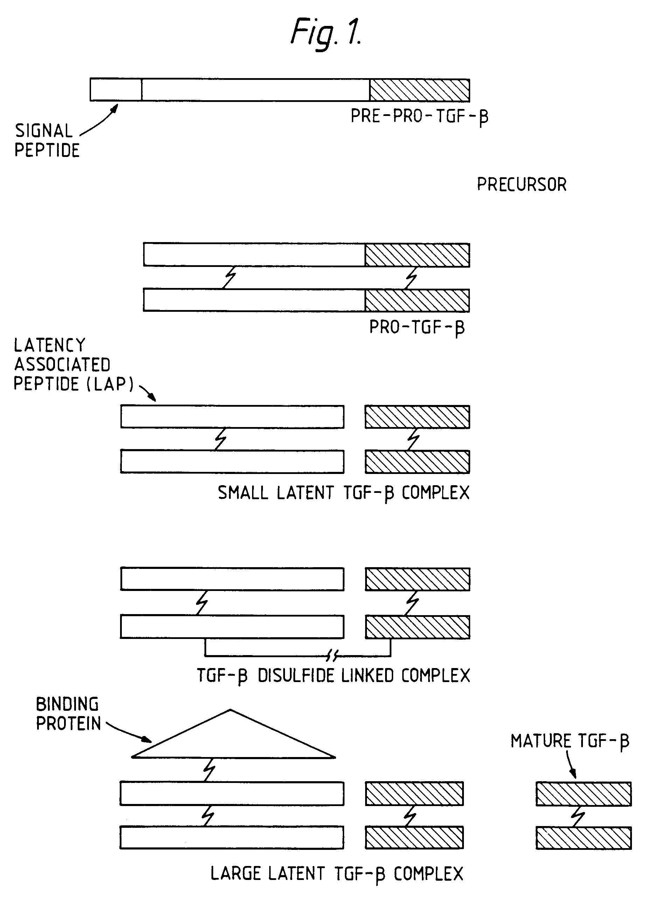

Figure 1 discloses latent forms of TGF-β and their standardized nomenclature. The full length preproTGF-β, whether in monomeric or dimeric form, is designated as the "precursor." The term "latency associated peptide" is assigned to that portion of the recombinantly produced latent TGF-β complex lacking the mature TGF-β (where the recombinant complexes include a "small latent TGF-β complex" and a "TGF-β disulfide-linked complex"). The 125-160 Kd binding protein that is disulfide linked to the latency associated peptide in the "large latent TGF-β complex" produced *in vivo* is designated as the "binding protein."

Figure 2 shows the construction of the vector pSVI-tPA used as a starting vector to construct the ultimate expression vector for latency associated peptide.

Figure 3 shows the construction of the vector pSVI2-tPA.

Figure 4 shows the construction of the vector pSVI3-tPA.

Figure 5 shows the construction of the vector pSVI5-tPA.

Figure 6 shows the construction of the vector pSVI6B-tPA.

Figure 7 shows the construction of the general expression vector pSVI6B5 into which the gene encoding latency associated peptide is conveniently inserted.

Figure 8 depicts the complete nucleotide and predicted amino acid sequence of the latency associated peptide. Predicted amino acids of the peptide are shown below the DNA sequence and are numbered from the first residue of the N-terminus of the peptide sequence. The mature TGF-β sequence is not included. Negative numbers indicate the signal sequence, and positive numbers designate the sequence of the secreted peptide.

Figure 9 depicts the construction of the expression vectors pSVI6B-LAP-ARG and pSVI6B-LAP-SER from pSVI6B5 used to transform mammalian host cells to produce the latency associated peptide.

Figure 10 illustrates the amount of bioactive TGF-β in ng/ml as determined by a mink lung fibroblast cell assay as a function of the degree of diluted heat-activated TGF-β supernatant (circles), and when heat-inactivated latency associated peptide (squares with dashed lines), or untreated latency associated peptide (squares with solid lines) is added to the heat-activated TGF-β.

Figure 11 depicts the sequences of human TGF-β1, human TGF-β2, human TGF-β3, chick TGF-β4, and frog TGF-β5.

### Description of the Preferred Embodiments

### A. Definitions

As used herein, "latency associated peptide," or "LAP," refers to a precursor portion of recombinantly produced TGF-β (i.e., any TGF-β in the family of TGF-βs) having a molecular weight of about 75 Kd (i.e., 75 up to about 78 Kd) when measured by SDS-PAGE on a non-reducing gel and having the amino acid sequence of Fig. 8, together with analogues and variants thereof having the biological activity of the corresponding recombinant LAP. The biological activity of recombinant LAP is shared by any analogue or variant thereof that is capable of antagonizing a biological activity of mature TGF-β. LAP that does not have the indicated biological activity, e.g., that is degraded by heat treatment, are excluded. The LAP is a dimeric molecule that forms a monomer on reduction having a molecular weight of about 37-39 Kd on reducing SDS-PAGE; the variants need not share this dimeric property.

Analogues or variants are defined as molecules in which the amino acid sequence, glycosylation, or other feature of recombinant LAP has been modified covalently or noncovalently. Thus, variants may or may not have a molecular weight of approximately 70 kD (as determined by SDS-PAGE carried out in the absence of a reducing agent such as, e.g., β-mercaptoethanol or dithiothreitol). For example, unglycosylated LAP having the recombinant LAP sequence will have a lower molecular weight on non-reducing SDS-PAGE. Amino acid sequence variants include not only alleles of the Fig. 8 sequence, but also predetermined mutations thereof. Generally, amino acid sequence variables have an amino acid sequence with at least about 80% sequence identity, and more typically at least about 90% sequence identity, to that of the recombinant LAP of Fig. 8. Henceforth, the term LAP shall mean either the recombinantly produced sequence, a variant form, or fragments representing biologically active site(s) within the molecule, unless otherwise appropriate.

Thus, included within the scope of the present invention is a LAP having the human recombinant LAP amino acid sequence as set forth in Figure 8, analogous LAP proteins from other species such as bovine, equine, porcine, ovine, canine, murine, feline LAP, and the like, LAP sequences from other types of TGF-β such as TGF-β2 (see, e.g., Madisen *et al.,* DNA, supra), and biologically active amino acid sequence variants of these LAP molecules, including alleles and *in vitro*-generated covalent derivatives of LAP that demonstrate its biological activity.

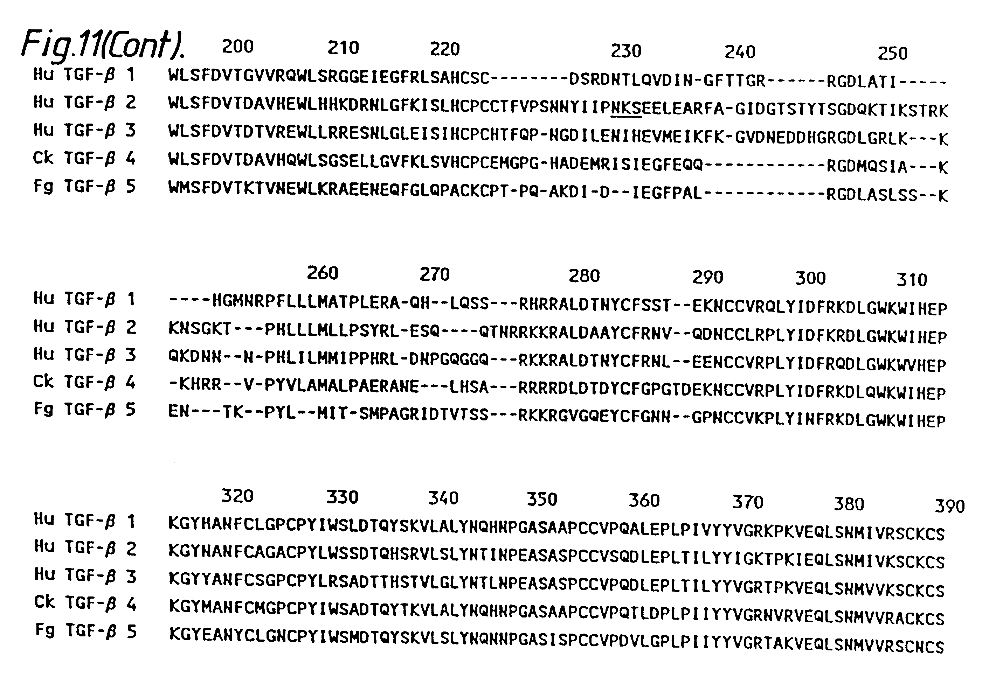

As used herein, the term "TGF-β" refers to the family of molecules described hereinabove that have the full-length, native amino acid sequence of any of the TGF-βs from any species. Reference to such TGF-β herein will be understood to be a reference to any one of the currently identified forms, including TGF-β1, TGF-β2, TGF-β3, TGF-β4, and TGF-β5 (whose sequences are shown in Figure 11), as well as to TGF-β species identified in the future, including polypeptides derived from the sequence of any known TGF-β and identical at 75% or more of the residues. The specific terms "TGF-β1," "TGF-β2," and "TGF-β3" refer to the TGF-βs defined in the literature, e.g., Derynck *et al.,* Nature, supra, Seyedin *et al*., J. Biol. Chem., 262, supra, and deMartin *et al*., supra.

Members of the TGF-β family are defined as those which have nine cysteine residues in the mature portion of the molecule, share at least 65% sequence identity with other known TGF-β sequences in the mature region, and may compete for the same receptor. In addition, they all appear to be encoded as a larger precursor that shares a region of high homology near the N-terminus and shows conservation of three cysteine residues in the portion of the precursor that will later be removed by processing. Moreover, the TGF-βs appear to have a processing site with four or five basic amino acids.

As used herein, "neutralization" of a TGF-β activity means neutralization of a TGF-β as measured by ³H-thymidine uptake inhibition of a mink lung fibroblast cell line, e.g., Mv-3D9 and CCL64.

The expression "antagonizing a biological activity of TGF-β" refers to blocking an activity of a TGF-β, e.g., substantially preventing at least one of the undesirable growth inhibitory, immunosuppressive, stroma forming (the stromal elements including inflammatory cells, endothelial cells, and fibroblasts), or anchorage-independent growth promoting activities of a mature TGF-β, as it is defined in the literature. The LAP is thus able to block the activity of all endogenously released TGF-β produced by tumors and suppressor lymphoid cells (T cells). Identification of LAP molecules having this effect is by means of the neutralization test detailed above.

Examples of conditions to be treated by therapy or prophylaxis using the processes and compositions of this invention include conditions caused by suppression of the immune system due to endogenous TGF-β production, including acquired immune deficiency syndrome, acute immune deficiencies resulting from severe injuries, burns, and illnesses such as viral or bacterial infections, cystic fibrosis, multi-organ systemic illnesses due to TGF-β production or overproduction, and TGF-β-producing tumors. Such conditions would not include those resulting from proliferation of cells of the lymphoid and myeloid lineages, which proliferation is inhibited by TGF-β (e.g., Burkitt's lymphoma, T-cell lymphomas, B-cell lymphomas, and various myelocytic leukemias).

Such conditions also include diseases caused by circulating TGF-β or TGF-β activated at a local site, such as those diseases associated with chronic granulation or hyperproliferation, such as fibrotic diseases, including fibrosis of any organ, e.g., scleroderma, and fibrosis of the lung, kidney, peritoneal cavity, and liver, and diseases caused by an excess of active TGF-β, such as vitreoretinopathy caused by an excess of TGF-β2. Also included is the prevention of illnesses and side effects caused by TGF-β and another regulator of immune function in a mammal as defined below.

The expression "regulators of immune function in a mammal" refers to cytokines and growth factors that regulate a mammal's immune function, including interleukins, tumor necrosis factor, lymphotoxin, epidermal growth factor, platelet-derived growth factor, TGF-α, macrophage migration inhibitory factor, macrophage activation factor, fibroblast growth factor, macrophage activating factors, interferons, and colony stimulating factors. These regulators may be derived from natural sources, formed by leukocytes, synthesized by chemical methods if appropriate, or prepared by recombinant techniques. Preferred of these are IL-1, IL-2, IL-6, and IFN-gamma.

### B. Modes of Carrying Out the Invention

### 1. Modifications of LAP

Derivatives and amino acid sequence variants of LAP are useful for their biological activity as it relates to therapeutic utility, as is set forth elsewhere herein.

### a. Covalent modification

Covalent modifications of a LAP molecule are included within the scope of this invention. Variant LAP fragments having up to about 100 residues may be conveniently prepared by *in vitro* synthesis. Such modifications may be introduced into the molecule by reacting targeted amino acid residues of the purified or crude protein with an organic derivatizing agent that is capable of reacting with selected side chains or terminal residues. The resulting covalent derivatives are useful in programs directed at identifying residues important for biological activity.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone,α-bromo-β-(5-imidozoyl)propionicacid,chloroacetylphosphate,N-alkylmaleimides, 3-nitro-2-pyridyl disulfide, methyl 2-pyridyl disulfide, p-chloromercuribenzoate,2-chloromercuri-4-nitrophenol,orchloro-7-nitrobenzo-2-oxa-1,3-diazole.

Histidyl residues are derivatized by reaction with diethylpyrocarbonate at pH 5.5-7.0 because this agent is relatively specific for the histidyl side chain. Para-bromophenacyl bromide also is useful; the reaction is preferably performed in 0.1M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues *per se* has been studied extensively, with particular interest in introducing spectral labels into tyrosyl residues by reaction with aromatic diazonium compounds or tetranitromethane. Most commonly, N-acetylimidizol and tetranitromethane are used to form O-acetyl tyrosyl species and 3-nitro derivatives, respectively. Tyrosyl residues are iodinated using ¹²⁵I or ¹³¹I to prepare labeled proteins for use in radioimmunoassay, the chloramine T method described above being suitable.

Carboxyl side groups (aspartyl or glutamyl) are selectively modified by reaction with carbodiimides (R'-N-C-N-R') such as 1-cyclohexyl-3-(2-morpholinyl-(4-ethyl) carbodiimide or 1-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains (T.E. Creighton, Proteins: Structure and Molecular Properties, W.H. Freeman & Co., San Francisco, pp. 79-86 [1983]), acetylation of the N-terminal amine, and, in some instances, amidation of the C-terminal carboxyl group.

### b. Mutation(s) in the DNA

Amino acid sequence variants of LAP can also be prepared by mutations in the DNA. Such variants include, for example, deletions from, or insertions or substitutions of, residues within the amino acid sequence shown in Figure 8. Any combination of deletion, insertion, and substitution may also be made to arrive at the final construct, provided that the final construct possesses the desired activity. Obviously, the mutations that will be made in the DNA encoding the variant must not place the sequence out of reading frame and preferably will not create complementary regions that could produce secondary mRNA structure (see EP 75,444A).

At the genetic level, these variants ordinarily are prepared by site-directed mutagenesis of nucleotides in the DNA encoding the LAP, thereby producing DNA encoding the variant, and thereafter expressing the DNA in recombinant cell culture. The variants typically exhibit the same qualitative biological activity as the naturally occurring analog.

While the site for introducing an amino acid sequence variation is predetermined, the mutation per se need not be predetermined. For example, to optimize the performance of a mutation at a given site, random mutagenesis may be conducted at the target codon or region and the expressed LAP variants screened for the optimal combination of desired activity. Techniques for making substitution mutations at predetermined sites in DNA having a known sequence are well known, for example, site-specific mutagenesis.

Preparation of LAP variants in accordance herewith is preferably achieved by site-specific mutagenesis of DNA that encodes an earlier prepared variant or a nonvariant version of the protein. Site-specific mutagenesis allows the production of LAP variants through the use of specific oligonucleotide sequences that encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 20 to 25 nucleotides in length is preferred, with about 5 to 10 residues on both sides of the junction of the sequence being altered. In general, the technique of site-specific mutagenesis is well known in the art, as exemplified by publications such as Adelman *et al*., DNA, 2: 183 (1983).

As will be appreciated, the site-specific mutagenesis technique typically employs a phage vector that exists in both a single-stranded and double-stranded form. Typical vectors useful in site-directed mutagenesis include vectors such as the M13 phage, for example, as disclosed by Messing *et al*., Third Cleveland Symposium on Macromolecules and Recombinant DNA, Editor A. Walton, Elsevier, Amsterdam (1981). These phage are readily commercially available and their use is generally well known to those skilled in the art. Alternatively, plasmid vectors that contain a single-stranded phage origin of replication (Veira *et al*., Meth. Enzymol., 153: 3 [1987]) may be employed to obtain single-stranded DNA.

In general, site-directed mutagenesis in accordance herewith is performed by first obtaining a single-stranded vector that includes within its sequence a DNA sequence that encodes the relevant protein. An oligonucleotide primer bearing the desired mutated sequence is prepared, generally synthetically, for example, by the method of Crea *et al*., Proc. Natl. Acad. Sci. (USA), 75: 5765 (1978). This primer is then annealed with the single-stranded protein-sequence-containing vector, and subjected to DNA-polymerizing enzymes such as E. coli polymerase I Klenow fragment, to complete the synthesis of the mutation-bearing strand. Thus, a heteroduplex is formed wherein one strand encodes the original non-mutated sequence and the second strand bears the desired mutation. This heteroduplex vector is then used to transform appropriate cells such as JM101 cells and clones are selected that include recombinant vectors bearing the mutated sequence arrangement.

After such a clone is selected, the mutated protein region may be removed and placed in an appropriate vector for protein production, generally an expression vector of the type that may be employed for transformation of an appropriate host.

### c. Types of Mutations

Amino acid sequence deletions generally range from about 1 to 30 residues, more preferably 1 to 10 residues, and typically are contiguous.

Amino acid sequence insertions include amino- and/or carboxyl-terminal fusions of from one residue to polypeptides of essentially unrestricted length, as well as intrasequence insertions of single or multiple amino acid residues. Intrasequence insertions (i.e., insertions within the mature LAP sequence) may range generally from about 1 to 10 residues, more preferably 1 to 5.

The third group of variants are those in which at least one amino acid residue in the LAP molecule, and preferably only one, has been removed and a different residue inserted in its place. Such substitutions preferably are made in accordance with the following Table 1 when it is desired to modulate finely the characteristics of a LAP molecule.

**Table 1**

| Original Residue | Exemplary Substitutions |
|---|---|
| Ala (A) | gly; ser |
| Arg (R) | lys |
| Asn (N) | gln; his |
| Asp (D) | glu |
| Cys (C) | ser |
| Gln (Q) | asn |
| Glu (E) | asp |
| Gly (G) | ala; pro |
| His (H) | asn; gln |
| Ile (I) | leu; val |
| Leu (L) | ile; val |
| Lys (K) | arg; gln; glu |
| Met (M) | leu; tyr; ile |
| Phe (F) | met; leu; tyr |
| Ser (S) | thr |
| Thr (T) | ser |
| Trp (W) | tyr |
| Tyr (Y) | trp; phe |
| Val (V) | ile; leu |

Substantial changes in function or immunological identity are made by selecting substitutions that are less conservative than those in Table 1, i.e., selecting residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions that in general are expected to produce the greatest changes in LAP properties will be those in which (a) glycine and/or proline (P) is substituted by another amino acid or is deleted or inserted; (b) a hydrophilic residue, e.g., seryl or threonyl, is substituted for (or by) a hydrophobic residue, e.g., leucyl, isoleucyl, phenylalanyl, valyl, or alanyl; (c) a cysteine residue is substituted for (or by) any other residue; (d) a residue having an electropositive side chain, e.g., lysyl, arginyl, or histidyl, is substituted for (or by) a residue having an electronegative charge, e.g., glutamyl or aspartyl; or (e) a residue having a bulky side chain, e.g., phenylalanine, is substituted for (or by) one not having such a side chain, e.g., glycine.

Most deletions and insertions, and substitutions in particular, are not expected to produce radical changes in the characteristics of the LAP molecule. However, when it is difficult to predict the exact effect of the substitution, deletion, or insertion in advance of doing so, one skilled in the art will appreciate that the effect will be evaluated by routine screening assays. For example, a variant typically is made by site-specific mutagenesis of the native LAP-encoding nucleic acid, expression of the variant nucleic acid in recombinant cell culture, and, optionally, purification from the cell culture, for example, by immunoaffinity adsorption on a rabbit polyclonal anti-LAP column (to absorb the variant by binding it to at least one remaining immune epitope).

Since LAP may aggregate into dimers, it is within the scope hereof to provide hetero- and homodimers, wherein one or both subunits are variants. Where both subunits are variants, the changes in amino acid sequence can be the same or different for each subunit chain. Heterodimers are readily produced by cotransforming host cells with DNA encoding both subunits and, if necessary, purifying the desired heterodimer, or by separately synthesizing the subunits, dissociating the subunits (e.g., by treatment with a chaotropic agent such as urea, guanidine hdyrochloride, or the like), mixing the dissociated subunits, and then reassociating the subunits by dialyzing away the chaotropic agent.

The activity of the cell lysate or purified LAP variant is then screened in a suitable screening assay for the desired characteristic. For example, changes in the level of TGF-β activity by the candidate mutants are measured by the appropriate assay. Modifications of such protein properties as redox or thermal stability, hydrophobicity, susceptibility to proteolytic degradation, or the tendency to aggregate with carriers or into multimers are assayed by methods well known to the ordinarily skilled artisan.

### 3. Recombinant Expression

The LAP molecule desired may be prepared by any technique, including recombinant methods. Likewise, an isolated DNA is understood herein to mean chemically synthesized DNA, cDNA, chromosomal, or extrachromosomal DNA with or without any 5'-flanking region. Preferably, the desired LAP herein is made by synthesis in recombinant cell culture.

For such synthesis, it is first necessary to secure nucleic acid that encodes a LAP. DNA encoding a LAP molecule may be obtained from a source of cells for human TGF-β by (a) preparing a cDNA library from these cells, (b) conducting hybridization analysis with labeled DNA encoding TGF-β or fragments thereof (up to or more than 100 base pairs in length) to detect clones in the library containing homologous sequences, and (c) analyzing the clones by restriction enzyme analysis and nucleic acid sequencing to identify full-length clones. DNA that is capable of hybridizing to a TGF-β-encoding DNA under low stringency conditions is useful for identifying DNA encoding TGF-β. Both high and low stringency conditions are defined further below. If full-length clones are not present in a cDNA library, then appropriate fragments may be recovered from the various clones using the nucleic acid sequence information disclosed herein and ligated at restriction sites common to the clones to assemble a full-length clone encoding the TGF-β. Alternatively, genomic libraries will provide the desired DNA. The mature portion of the TGF-β is cleaved and the remaining precursor (LAP) portion is the portion employed herein. The sequence of the DNA encoding human LAP that was ultimately determined is shown in Fig. 8. Once this DNA has been identified and isolated from the library it is ligated into a replicable vector for further cloning or for expression.

In one example of a recombinant expression system a LAP-encoding gene is expressed in mammalian cells by transformation with an expression vector comprising DNA encoding the LAP. It is preferable to transform host cells capable of accomplishing such processing so as to obtain the LAP in the culture medium or periplasm of the host cell, i.e., obtain a secreted molecule.

### a. Useful Host Cells and Vectors

The vectors and methods disclosed herein are suitable for use in host cells over a wide range of prokaryotic and eukaryotic organisms.

In general, prokaryotes are preferred for initial cloning, amplifying, or storing the vectors of interest. Vector DNA is easily obtainable from certain prokaryotes. E. coli K12 strain MM 294 (ATCC No. 31,446) is particularly useful for this purpose. Other microbial strains that may be used include E. coli strains such as E. coli B and E. coli X1776 (ATCC No. 31,537). These examples are, of course, intended to be illustrative rather than limiting.

Prokaryotes may also be used for expression. The aforementioned strains, as well as E. coli strains W3110 (F, lambda-, prototrophic, ATCC No. 27,325), K5772 (ATCC No. 53,635), and SR101, bacilli such as Bacillus subtilis, and other enterobacteriaceae such as Salmonella typhimurium or Serratia marcesans, and various pseudomonas species, may be used.

In general, plasmid vectors containing replicon and control sequences that are derived from species compatible with the host cell are used in connection with these prokaryotic hosts. The vector ordinarily carries a replication site, as well as marking sequences that are capable of providing phenotypic selection in transformed cells. For example, E. coli is typically transformed using pBR322, a plasmid derived from an E. coli species (see, e.g., Bolivar *et al*., Gene, 2: 95 [1977]). pBR322 contains genes for ampicillin and tetracycline resistance and thus provides easy means for identifying transformed cells. The pBR322 plasmid, or other microbial plasmid or phage, must also contain, or be modified to contain, promoters that can be used by the microbial organism for expression of the selectable marker genes.

Those promoters most commonly used in recombinant DNA construction include the β-lactamase (penicillinase) and lactose promoter systems (Chang *et al*., Nature, 375: 615 [1978]; Itakura *et al*., Science, 198: 1056 [1977]; Goeddel *et al*, Nature, 281: 544 [1979]) and a tryptophan (trp) promoter system (Goeddel *et al*., Nucleic Acids Res., 8 4057 [1980]; EPO Appl. Publ. No. 0036,776). While these are the most commonly used, other microbial promoters have been discovered and utilized, and details concerning their nucleotide sequences have been published, enabling a skilled worker to ligate them functionally with plasmid vectors (see, e.g., Siebenlist *et al*., Cell, 20: 269 [1980]).

In addition to prokaryotes, eukaryotic microbes, such as yeast cultures, may also be used. Saccharomyces cerevisiae, or common baker's yeast, is the most commonly used among eukaryotic microorganisms, although a number of other strains are commonly available. For expression in Saccharomyces, the plasmid YRp7, for example (Stinchcomb *et al*, Nature, 282: 39 [1979]; Kingsman *et al*., Gene, 7: 141 [1979]; Tschemper *et al*., Gene, 10: 157 [1980]), is commonly used. This plasmid already contains the trp1 gene that provides a selection marker for a mutant strain of yeast lacking the ability to grow in tryptophan, for example, ATCC No. 44,076 or PEP4-1 (Jones, Genetics, 85: 12 [1977]). The presence of the trp1 lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzeman *et al*., J. Biol. Chem., 255: 2073 [1980]) or other glycolytic enzymes (Hess *et al.*, J. Adv. Enzyme Reg., 7: 149 [1968]; Holland *et al.*, Biochemistry, 17:4900 [1978]), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination. Other promoters, which have the additional advantage of transcription controlled by growth conditions, are the promoter region for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate cells in culture (tissue culture) has become a routine procedure in recent years [Tissue Culture, Academic Press, Kruse and Patterson, editors (1973)].

Examples of such useful host cell lines include monkey kidney CVI line transformed by SV40 sequences (COS-7, ATCC CRL 1651); human embryonic kidney line (293, Graham *et al*., J. Gen. Virol., 36: 59 [1977]); baby hamster kidney cells (BHK, ATCC CCL 10); Chinese hamster ovary cells (Urlaub and Chasin, Proc. Natl. Acad. Sci. USA, 77: 4216 [1980]); mouse sertoli cells (TM4, Mather, Biol. Reprod., 23: 243-251 [1980]); monkey kidney cells (CVI, ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor cells (MMT 060562, ATCC CCL51); rat hepatoma cells (HTC, M1.54, Baumann *et al*., J. Cell. Biol., 85: 1-8 [1980]); and TRI cells (Mather *et al*., Annals N.Y. Acad. Sci., 383: 44-68 [1982]). The most preferred eukaryotic host herein for stable expression is a Chinese hamster ovary cell line.

For use in mammalian cells, the control functions on the expression vectors are often provided by viral material. For example, commonly used promoters are derived from the genomes of polyoma, Adenovirus2, retroviruses, cytomegalovirus, and most frequently Simian Virus 40 (SV40). Other promoters are those from heterologous sources, e.g., the beta actin promoter. The early and late promoters of SV40 virus are particularly useful because both are obtained easily from the virus as a fragment that also contains the SV40 viral origin of replication [Fiers *et al.*, Nature, 273: 113 (1978)]. Smaller or larger SV40 fragments may also be used, provided there is included the approximately 250-bp sequence extending from the HindIII site toward the BglI site located in the viral origin of replication. The immediate early promoter of the human cytomegalovirus is conveniently obtained as a HindIII restriction fragment. Greenawa*y et al*., Gene, 18: 355-360 (1982). Further, it is also possible, and often desirable, to utilize promoter or control sequences normally associated with the desired gene sequence, provided such control sequences are compatible with the host cell systems.

Transcription of a DNA encoding the LAP by higher eukaryotes is increased by inserting an enhancer sequence into the vector. The enhancer is a cis-acting element of DNA, usually about from 10 to 300 bp, that acts on a promoter to enhance its transcription-initiation activity. Enhancers are relatively orientation and position independent, having been found 5' (Laimins *et al*., Proc. Natl. Acad. Sci. USA, 78: 993 [1981]) and 3' (Lusky *et al*., Mol. Cell Bio., 3: 1108 [1983]) to the transcription unit, within an intron (Banerji *et al.*, Cell, 33: 729 [1983]) as well as within the coding sequence itself (Osborne *et al*., Mol. Cell Bio., 4: 1293 [1984]). Preferably, however, the enhancer element is located upstream of the promoter sequence for this invention. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. Most preferred herein is the SV40 enhancer region.

Expression vectors used in mammalian host cells will also contain polyadenylation sites. Examples of polyadenylation regions are those derived from viruses such as, e.g., the SV40 (early and late) or HBV.

An origin of replication may be provided either by construction of the vector to include an exogenous origin, such as may be derived from SV40 or other viral (e.g., Polyoma, Adeno, VSV, BPV) source, or may be provided by the host cell. If the vector is integrated into the host cell chromosome, the latter is often sufficient.

The expression vectors may suitably contain a selection gene, also termed a selectable marker. A selection gene encodes a protein necessary for the survival or growth of a host cell transformed with the vector. Examples of suitable selectable markers for mammalian cells include dihydrofolate reductase (DHFR), thymidine kinase (TK), or neomycin. When such selectable markers are successfully transferred into a mammalian host cell, the transformed mammalian host cell can survive if placed under selective pressure.

There are two widely used distinct categories of selective regimes. The first category is based on the metabolism of a cell and the use of a mutant cell line that lacks the ability to grow independent of a supplemented medium. Two examples are CHO DHFR⁻cells and mouse LTK⁻cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented medium. An alternative to supplementing the medium is to introduce an intact DHFR or TK gene into cells lacking the respective genes, thus altering their growth requirements. Individual cells that were not transformed with the DHFR or TK gene will not be capable of survival in non-supplemented medium. Therefore, direct selection of those cells requires cell growth in the absence of supplemental nutrients.

The second category is dominant selection, which refers to a selection scheme that does not require the use of a mutant cell line. This method typically employs a drug to arrest growth of a host cell. Those cells that have a novel gene would express a protein conveying drug resistance and would survive the selection. Examples of drugs used in dominant selection include neomycin (Southern and Berg, J. Molec. Appl. Genet., 1: 327 [1982]), mycophenolic acid (Mulligan and Berg, Science, 209: 1422 [1980]), or hygromycin (Sugden *et al.*, Mol. Cell. Biol., 5: 410-413 [1985]). The three examples given above employ bacterial genes under eukaryotic control to convey resistance to the appropriate drug, i.e., neomycin (G418 or geneticin), xgpt (mycophenolic acid), or hygromycin, respectively.

Satisfactory amounts of protein are produced by cell cultures; however, refinements, using a secondary coding sequence, serve to enhance production levels even further. One secondary coding sequence comprises dihydrofolate reductase (DHFR) that is affected by an externally controlled parameter, such as methotrexate (MTX), thus permitting control of expression by control of the methotrexate concentration.

In selecting a preferred host cell for transfection by the vectors of the invention that comprise DNA sequences encoding both LAP and DHFR protein, it is appropriate to select the host according to the type of DHFR protein employed. If wild-type DHFR protein is employed, it is preferable to select a host cell that is deficient in DHFR, thus permitting the use of the DHFR coding sequence as a marker for successful transfection in selective medium that lacks hypoxanthine, glycine, and thymidine. An appropriate host cell in this case is the Chinese hamster ovary (CHO) cell line deficient in DHFR activity, prepared and propagated as described by Urlaub and Chasin, Proc. Natl. Acad. Sci. (USA) 77: 4216 1980).

On the other hand, if DHFR protein with low binding affinity for MTX is used as the controlling sequence, it is not necessary to use DHFR-deficient cells. Because the mutant DHFR is resistant to methotrexate, MTX-containing media can be used as a means of selection provided that the host cells are themselves methotrexate sensitive. Most eukaryotic cells that are capable of absorbing MTX appear to be methotrexate sensitive. One such useful cell line is a CHO line, CHO-K1 (ATCC No. CCL 61). Extremely good amounts of polypeptide are produced by cell cultures using the method of this invention; however, refinements, using cotransfection with a separate vector encoding a secondary coding sequence. One secondary coding sequence comprises dihydrofolate reductase (DHFR) that is affected by an externally controlled parameter, such as methotrexate (MTX), thus permitting control of expression by control of the methotrexate concentration.

### b. Typical Methodology Employable

Construction of suitable vectors containing the desired coding and control sequences employs standard recombinant techniques. Isolated plasmids or DNA fragments are cleaved, tailored, and religated to form the desired plasmid.

If flush ends are required, the cleaved DNA preparation may be treated for 30 minutes at 37°C with DNA Polymerase I (Klenow fragment) or T4 DNA polymerase, phenol-chloroform extracted, and ethanol precipitated. 3' protruding ends are removed by the 3' to 5' exonucleolytic activity of either enzyme, and the 5' protruding ends are made flush by the 5' to 3' polymerase activity incorporating complementary nt until the end of the fragment is reached.

Size separation of the cleaved fragments may be performed using 6 percent polyacrylamide gel described by Goeddel *et al*., Nucleic Acids Res., 8: 4057 (1980).

For analysis to confirm correct sequences in plasmids constructed, the ligation mixtures are typically used to transform E. coli K12 strain 294 (ATCC 31,446) or other suitable E. coli strains, and successful transformants selected by ampicillin or tetracycline resistance where appropriate. Plasmids from the transformants are prepared and analyzed by restriction mapping and/or DNA sequencing by the method of Messing *et al*., Nucleic Acids Res., 9: 309 (1981) or by the method of Maxam *et al*., Meth. Enzym, 65: 499 (1980).

After introduction of the DNA into the mammalian cell host and selection in medium for stable transfectants, amplification of DHFR-protein-coding sequences is effected by growing host cell cultures in the presence of approximately 200-500 nM concentrations of methotrexate, a competitive inhibitor of DHFR activity. The effective range of concentration is highly dependent, of course, upon the nature of the DHFR gene and the characteristics of the host. Clearly, generally defined upper and lower limits cannot be ascertained. Suitable concentrations of other folic acid analogs or other compounds that inhibit DHFR could also be used. MTX itself is, however, convenient, readily available, and effective.

Other techniques employable are described in a section just prior to the examples.

### 4. Utilities and Formulation

The LAP molecules herein have a number of therapeutic uses associated with the antagonism or neutralization of TGF-β activity. Such uses include the treatment of fibrotic diseases and patients who are immunosuppressed as measured by the ability to contract and sustain a secondary infection for a prolonged period of time. Particularly of interest are patients suffering from recurrent or chronic viral, bacterial, mycological, or parasitic infections. Other physiological conditions that could be improved based on the ability of LAP to bind reversibly to mature TGF-β are also included herein.

Furthermore, LAP can be used as a means of preventing side effects of other immune regulators (e.g., recombinant cytokines) when they are used as pharmaceuticals or used in combination with such regulators, depending on the particular clinical scenario.

For the indications referred to above, the LAP molecule will be formulated and dosed in a fashion consistent with good medical practice taking into account the specific disorder to be treated, the condition of the individual patient, the site of delivery of the LAP, the method of administration, and other factors known to practitioners. Thus, for purposes herein, the "therapeutically effective amount" of the LAP is an amount that is effective either to prevent, lessen the worsening of, alleviate, or cure the treated condition, in particular that amount which is sufficient to antagonize the activity of a TGF-β *in vivo*.

The LAP is prepared for storage or administration by mixing LAP having the desired degree of purity with physiologically acceptable carriers, excipients, or stabilizers. Such materials are non-toxic to recipients at the dosages and concentrations employed. If the LAP is water soluble, it may be formulated in a buffer such as phosphate or other organic acid salt preferably at a pH of about 7 to 8. If a LAP variant is only partially soluble in water, it may be prepared as a microemulsion by formulating it with a nonionic surfactant such as Tween, Pluronics, or PEG, e.g., Tween 80, in an amount of 0.04-0.05% (w/v), to increase its solubility.

Optionally other ingredients may be added such as antioxidants, e.g., ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; and sugar alcohols such as mannitol or sorbitol.

The LAP to be used for therapeutic administration must be sterile. Sterility is readily accomplished by filtration through sterile filtration membranes (e.g., 0.2 micron membranes). The LAP ordinarily will be stored in lyophilized form or as an aqueous solution if it is highly stable to thermal and oxidative denaturation. The pH of the LAP preparations typically will be about from 6 to 8, although higher or lower pH values may also be appropriate in certain instances. It will be understood that use of certain of the foregoing excipients, carriers, or stabilizers will result in the formation of salts of the LAP.

If the LAP is to be used parenterally, therapeutic compositions containing the LAP generally are placed into a container having a sterile access port, for example, an intravenous solution bag or vial having a stopper pierceable by a hypodermic injection needle.

Generally, where the disorder permits, one should formulate and dose the LAP for site-specific delivery. This is convenient in the case of fibrotic diseases and tumors.

Sustained release formulations may also be prepared, and include the formation of microcapsular particles and implantable articles. For preparing sustained-release LAP compositions, the LAP is preferably incorporated into a biodegradable matrix or microcapsule. A suitable material for this purpose is a polylactide, although other polymers of poly-(α-hydroxycarboxylic acids), such as poly-D-(-)-3-hydroxybutyric acid (EP 133,988A), can be used. Other biodegradable polymers include poly(lactones), poly(acetals), poly(orthoesters), or poly(orthocarbonates). The initial consideration here must be that the carrier itself, or its degradation products, is nontoxic in the target tissue and will not further aggravate the condition. This can be determined by routine screening in animal models of the target disorder or, if such models are unavailable, in normal animals.

For examples of sustained release compositions, see U.S. Patent No. 3,773,919, EP 58,481A, U.S. Patent No. 3,887,699, EP 158,277A, Canadian Patent No. 1176565, U. Sidman *et al*., "Biopolymers" 22:547 [1983], and R. Langer *et al*., "Chem. Tech." 12:98 [1982].

When applied topically, the LAP is suitably combined with other ingredients, such as carriers and/or adjuvants. There are no limitations on the nature of such other ingredients, except that they must be pharmaceutically acceptable and efficacious for their intended administration, and cannot degrade the activity of the active ingredients of the composition. Examples of suitable vehicles include ointments, creams, gels, or suspensions, with or without purified collagen. The compositions also may be impregnated into transdermal patches, plasters, and bandages, preferably in liquid or semi-liquid form.

For obtaining a gel formulation, the LAP formulated in a liquid composition may be mixed with an effective amount of a water-soluble polysaccharide or synthetic polymer such as polyethylene glycol to form a gel of the proper viscosity to be applied topically. The polysaccharide that may be used includes, for example, cellulose derivatives such as etherified cellulose derivatives, including alkyl celluloses, hydroxyalkyl celluloses, and alkylhydroxyalkyl celluloses, for example, methylcellulose, hydroxyethyl cellulose, carboxymethyl cellulose, hydroxypropyl methylcellulose, and hydroxypropyl cellulose; starch and fractionated starch; agar; alginic acid and alginates; gum arabic; pullullan; agarose; carrageenan; dextrans; dextrins; fructans; inulin; mannans; xylans; arabinans; chitosans; glycogens; glucans; and synthetic biopolymers; as well as gums such as xanthan gum; guar gum; locust bean gum; gum arabic; tragacanth gum; and karaya gum; and derivatives and mixtures thereof. The preferred gelling agent herein is one that is inert to biological systems, nontoxic, simple to prepare, and not too runny or viscous, and will not destabilize the LAP held within it.

Preferably the polysaccharide is an etherified cellulose derivative, more preferably one that is well defined, purified, and listed in USP, e.g., methylcellulose and the hydroxyalkyl cellulose derivatives, such as hydroxypropyl cellulose, hydroxyethyl cellulose, and hydroxypropyl methylcellulose. Most preferred herein is methylcellulose.

The polyethylene glycol useful for gelling is typically a mixture of low and high molecular weight polyethylene glycols to obtain the proper viscosity. For example, a mixture of a polyethylene glycol of molecular weight 400-600 with one of molecular weight 1500 would be effective for this purpose when mixed in the proper ratio to obtain a paste.

The term "water soluble" as applied to the polysaccharides and polyethylene glycols is meant to include colloidal solutions and dispersions. In general, the solubility of the cellulose derivatives is determined by the degree of substitution of ether groups, and the stabilizing derivatives useful herein should have a sufficient quantity of such ether groups per anhydroglucose unit in the cellulose chain to render the derivatives water soluble. A degree of ether substitution of at least 0.35 ether groups per anhydroglucose unit is generally sufficient. Additionally, the cellulose derivatives may be in the form of alkali metal salts, for example, the Li, Na, K, or Cs salts.

If methylcellulose is employed in the gel, preferably it comprises about 2-5%, more preferably about 3%, of the gel and the LAP is present in an amount of about 300-1000 »g per ml of gel.

The dosage of LAP to be employed is dependent upon the factors described above, especially the type of disease being treated. As a general proposition, a dose of about 0.015 to 15 mg/kg of LAP may be administered to the patient, whether via, e.g., one or more single administrations, continuous infusion, or bolus injection. For example, an initial dose of the LAP is administered to the patient by injection or infusion. For repeated administrations over several days or longer, depending on the condition, the treatment is repeated until a desired suppression of disease symptoms occurs. However, other dosage regimens may be useful.

According to another embodiment of the invention, the effectiveness of the LAP may be improved by administering it serially or in combination with another agent that is effective for this purpose, such as one or more anti-TGF-β antibodies, a regulator of immune function, as defined above, or one or more conventional therapeutic agents such as, for example, alkylating agents, folic acid antagonists, anti-metabolites of nucleic acid metabolism, spindle poisons, antibiotics, pyrimidine analogs, 5-FU, purine nucleosides, amines, amino acids, triazol nucleosides, corticosteroids, calcium, retinoids, lipoxygenase and cyclooxygenase inhibitors, fumaric acid and its salts, analgesics, psychopharmaceuticals, local anesthetics, spasmolytics, and beta-blockers. See, for example, Foley, N. Eng. J. Med., 313: 84-95 (1985), Nicholson, Lancet, ii, 503-506, 562-564, 617-621, 677-682, and 736-739 (1984), and DeClerq, Biochem. J., 205:1-13 (1982). Such other agents may be present in the composition being administered or may be administered separately.

Alternatively, the LAP molecule is suitably administered serially or in combination with radiological treatments--irradiation or introduction of radioactive substances--such as those referred to in UICC (ed.) in Klinische Onkologie, Springer-Verlag (1982).

In a still further embodiment, the LAP is used in combination with a mature TGF-β, whether heterogeneous or homogeneous with the LAP. For example, a LAP from TGF-β1 can be employed with mature TGF-β1, TGF-β2, or TGF-β3. The relative amounts of mature TGF-β to LAP are generally about equimolar.

The mature TGF-β is administered simultaneously with the LAP, whether in the same formulation or in separate administrations to the same locale such as a vein. Preferably, the TGF-β and LAP are mixed together before administration. If the complex is unstable, the ingredients are mixed together in approximately equimolar amounts just before administration. If the complex is stable, the TGF-β and LAP are mixed together and the inactive complex is purified away from the reaction mixture and the purified material is administered. Preferably, the administration is systemic, because the advantage resides in the reduction of side effects associated with certain doses of systemically administered TGF-β.

The combination of mature TGF-β with LAP can be used by any process enhanced by the pool of latent TGF-β, including soft and hard tissue generation, such as wound healing and bone formation, and immunosuppression. The therapy applies to surgical as well as non-surgical intervention; for example, the combination can be given to a patient a day before surgery so that tissue strength at the wound site will be increased, or given to a patient with a simple bone fracture to increase the bone healing rate.

Finally, the LAP is suitably used in a diagnostic assay to capture active TGF-β in the serum of a patient suffering from a condition, including a disease, that can be diagnosed by detecting mature TGF-β in serum. This technique is particularly suitable for sensitive detection of minute amounts of mature TGF-β in the serum. Specifically, the method comprises adding to the serum, whether *in vitro* or *in vivo*, LAP that has been labeled by any suitable detectable moiety, such as ³²P or alkaline phosphatase, and assaying for the presence of labeled complexes of LAP and mature TGF-β in the serum.

In order to simplify the examples and claims, certain frequently occurring methods will be referenced by shorthand phrases.

"Transfection" refers to the taking up of an expression vector by a host cell whether or not any coding sequences are in fact expressed. Numerous methods of transfection are known to the ordinarily skilled artisan, for example, CaPO₄ and electroporation. Successful transfection is generally recognized when any indication of the operation of this vector occurs within the host cell.

"Transformation" means introducing DNA into an organism so that the DNA is replicable, either as an extrachromosomal element or by chromosomal integrant. Depending on the host cell used, transformation is done using standard techniques appropriate to such cells. The calcium treatment employing calcium chloride, as described by Cohen, S.N. Proc. Natl. Acad. Sci. (USA), 69: 2110 (1972); Mandel *et al*., J. Mol. Biol. 53:154 (1970); and more recently Liljestrom *et al*., Gene, 40: 241-246 (1985), is generally used for prokaryotes or other cells that contain substantial cell-wall barriers. For mammalian cells without such cell walls, the calcium phosphate precipitation method of Graham, F. and van der Eb, A., Virology, 52: 456-457 (1978) is preferred. General aspects of mammalian cell host system transformations have been described by Axel in U.S. Pat. No. 4,399,216 issued August 16, 1983. Transformations into yeast are typically carried out according to the method of Van Solingen, P., *et al*., J. Bact., 130: 946 (1977) and Hsiao, C.L., *et al*., Proc. Natl. Acad. Sci. (USA) 76: 3829 (1979). However, other methods for introducing DNA into cells such as by nuclear injection or by protoplast fusion may also be used.

"Operably linked" refers to juxtaposition such that the normal function of the components can be performed. Thus, a coding sequence "operably linked" to control sequences refers to a configuration wherein the coding sequence can be expressed under the control of these sequences and wherein the DNA sequences being linked are contiguous and, in the case of a secretory leader, contiguous and in reading phase. For example, DNA for a presequence or secretory leader is operably linked to DNA for a polypeptide if it is expressed as a preprotein that participates in the secretion of the polypeptide; a promoter or enhancer is operably linked to a coding sequence if it effects the transcription of the sequence; or a ribosome binding site is operably linked to a coding sequence if it is positioned so as to facilitate translation. Linking is accomplished by ligation at convenient restriction sites. If such sites do not exist, then synthetic oligonucleotide adaptors or linkers are used in accord with conventional practice.

"Control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

"Expression system" refers to DNA sequences containing a desired coding sequence and control sequences in operable linkage, so that hosts transformed with these sequences are capable of producing the encoded proteins. To effect transformation, the expression system may be included on a vector; however, the relevant DNA may then also be integrated into the host chromosome.

As used herein, "cell," "cell line," and "cell culture" are used interchangeably and all such designations include progeny. Thus, "transformants" or "transformed cells" includes the initial transformant and cultures derived therefrom without regard for the number of transfers. It is also understood that all progeny may not be precisely identical in DNA content, due to deliberate or inadvertent mutations. Mutant progeny that have the same functionality as screened for in the originally transformed cell are included. Where distinct designations are intended, it will be clear from the context.

"Plasmids" are designated by a lower case p preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

The technique of "PCR" as used herein generally refers to the following: Minute amounts of a specific piece of DNA can be amplified using the polymerase chain reaction (PCR) as described in U.S. Pat. No. 4,683,195 issued July 28, 1987. Generally, sequence information from the ends of the stretch of interest or beyond needs to be available, such that oligonucleotide primers can be designed; these primers will point towards one another, and will be identical or similar in sequence to opposite strands of the template to be amplified. The 5' terminal nucleotides of the two primers will coincide with the ends of the amplified material. PCR can be used to amplify specific DNA sequences from total genomic DNA, cDNA transcribed from total cellular RNA, bacteriophage or plasmid sequences, etc. See generally H. Erlich, ed., PCR Technology, Stockton Press, NY, 1989.

The technique of "PCR mutagenesis" as used herein refers to the following (see Erlich, supra, the chapter by R. Higuchi, p. 61-70): When small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template. For introduction of a mutation into a plasmid DNA, one of the primers is designed to overlap the position of the mutation and to contain the mutation; the sequence of the other primer must be identical to a stretch of sequence of the opposite strand of the plasmid, but this sequence can be located anywhere along the plasmid DNA. It is preferred, however, that the sequence of the second primer is located within 200 nt from that of the first, such that in the end the entire amplified region of DNA bounded by the primers can be easily sequenced. PCR amplification using a primer pair like the one just described results in a population of DNA fragments that differ at the position of the mutation specified by the primer, and possibly at other positions, as template copying is somewhat error-prone.

In the procedure detailed below, the ratio of template to product material is extremely low, and as a result, the vast majority of product DNA fragments incorporate the desired mutation(s). This product material is used to replace the corresponding region in the plasmid that served as PCR template using standard DNA technology. Mutations at separate positions can be introduced simultaneously by either using a mutant second primer, or performing a second PCR with different mutant primers and ligating the two resulting PCR fragments simultaneously to the vector fragment in a three (or more)-part ligation.

The PCR mutagenesis procedure employed in the examples below was as follows: Template plasmid DNA (1 »g) was linearized by digestion with a restriction endonuclease that has a unique recognition site in the plasmid DNA outside of the region to be amplified. Of this material, 1-5 ng was added to a PCR mixture containing 16.6 mM (NH₄)₂SO₄, 67 mM Tris.HCl (pH 8.8), 6.7 mM MgCl₂, 6.7 »M EDTA, 10 mM 2-mercaptoethanol, 1 mM each dATP, dCTP, dGTP, and TTP, 170 »g/ml bovine serum albumin, 25 pmole of each oligonucleotide primer, and 1 »l Thermus aquaticus (Taq) DNA polymerase (5 units/»l, purchased from Perkin-Elmer Cetus, Norwalk, CT and Emeryville, CA) in a final volume of 50 »l in a 0.5-ml reaction vial. The reaction mixture was overlayed with 35 »l mineral oil and inserted into a DNA Thermal Cycler (purchased from Perkin-Elmer Cetus) programmed as follows:

| | |
|---|---|
| time-delay file | 12 min. 94°C |
| thermo-cycle file | 1 min. 50°C |
| | 2-3 min. 68-72°C |
| | 1 min. 94°C |
| | 20 cycles |
| time-delay file | 4 min. 50°C |
| time-delay file | 12 min. 68°C |
| soak file | 4°C |

Each file shown above was linked to the one on the next line. At the end of the program, the reaction vial was removed from the thermal cycler and the aqueous phase transferred to a new vial, extracted with phenol/chloroform/isoamylalcohol (50:50:1 vol), and ethanol precipitated, and the DNA was recovered by standard procedures. This material was subsequently subjected to the appropriate treatments for insertion into a vector.

"Digestion" of DNA refers to catalytic cleavage of the DNA with an enzyme that acts only at specific nt sequences in the DNA. Such enzymes are called restriction enzymes, and the sequence for which each is specific is called a restriction site. The various restriction enzymes used herein are commercially available and their reaction conditions, cofactors, and other requirements as established by the enzyme suppliers are used. Restriction enzymes commonly are designated by abbreviations composed of a capital letter followed by other letters representing the microorganism from which each restriction enzyme originally was obtained and then a number designating the particular enzyme. In general, about 1 »g of plasmid or DNA fragment is used with about 1-2 units of enzyme in about 20 »l of buffer solution. Appropriate buffers and substrate amounts for particular restriction enzymes are specified by the manufacturer. Incubation of about 1 hour at 37°C is ordinarily used, but may vary in accordance with the supplier's instructions. After incubation, protein is removed by extraction with phenol and chloroform, and the digested nucleic acid is recovered from the aqueous fraction by precipitation with ethanol. When appropriate, digestion with a restriction enzyme is followed by bacterial alkaline phosphatase-mediated hydrolysis of the terminal 5' phosphates to prevent the two ends of a DNA fragment from "circularizing" or forming a closed loop that would impede insertion of another DNA fragment at the restriction site. Unless otherwise stated, digestion of plasmids is not followed by 5' terminal dephosphorylation. Procedures and reagents for dephosphorylation are conventional (T. Maniatis *et al*., 1982, Molecular Clonine: A Laboratory Manual (New York: Cold Spring Harbor Laboratory, 1982) pp. 133-134).

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see R. Lawn *et al*., Nucleic Acids Res. 9:6103-6114 (1981), and D. Goeddel *et al*., Nucleic Acids Res. 8:4057 (1980).

"Ligation" refers to the process of forming phosphodiester bonds between two double stranded nucleic acid fragments (T. Maniatis *et al*., 1982, supra, p. 146). Unless otherwise provided, ligation may be accomplished using known buffers and conditions with 10 units of T4 DNA ligase ("ligase") per 0.5 »g of approximately equimolar amounts of the DNA fragments to be ligated.

"Preparation" of DNA from transformants means isolating plasmid DNA from microbial culture. Unless otherwise provided, the alkaline/SDS method of Maniatis *et al*., 1982, supra, p. 90, may be used.

"Oligonucleotides" are short-length, single- or double-stranded polydeoxynucleotides that are chemically synthesized by known methods (such as phosphotriester, phosphite, or phosphoramidite chemistry, using solid phase techniques such as described in EP Pat. Pub. No. 266,032 published May 4, 1988, or via deoxynucleoside H-phosphonate intermediates as described by Froehler *et al*., Nucl. Acids Res., 14: 5399-5407 [1986]). They are then purified on polyacrylamide gels.

The following examples are intended to illustrate the best mode now known for practicing the invention, but the invention is not to be considered limited thereto.

### EXAMPLE 1

### Cloning and Expression of DNA encoding LAP

### 1. Construction of t-PA Expression Vectors

Various t-PA vectors were derived from parent vector pSVI-tPA by altering a number of features of the splice donor-intron-splice acceptor unit.

### a. pSVI-tPA

Portions of two previously disclosed mammalian expression vectors, pRK-tPA and pE348DHFRUC, were combined to generate pSVI-tPA.

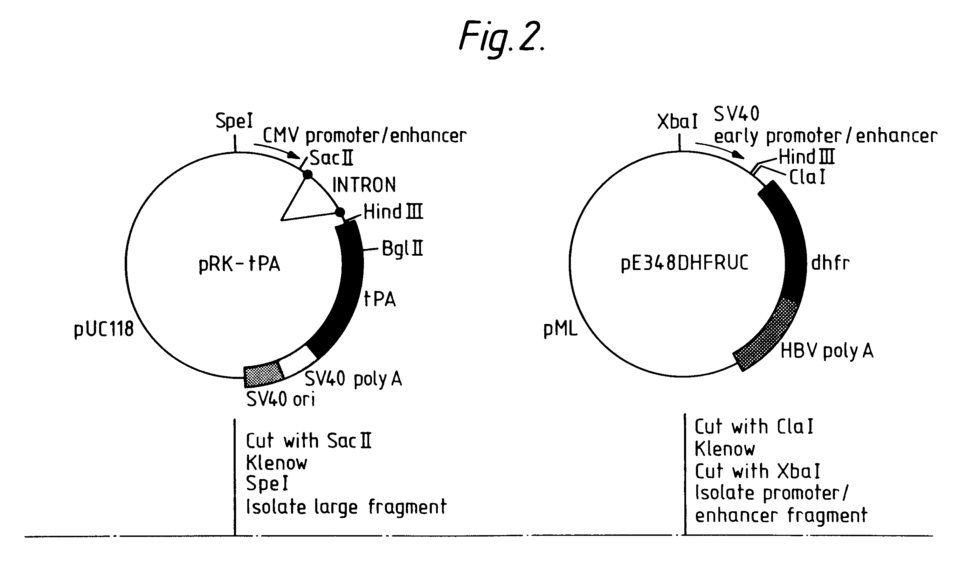

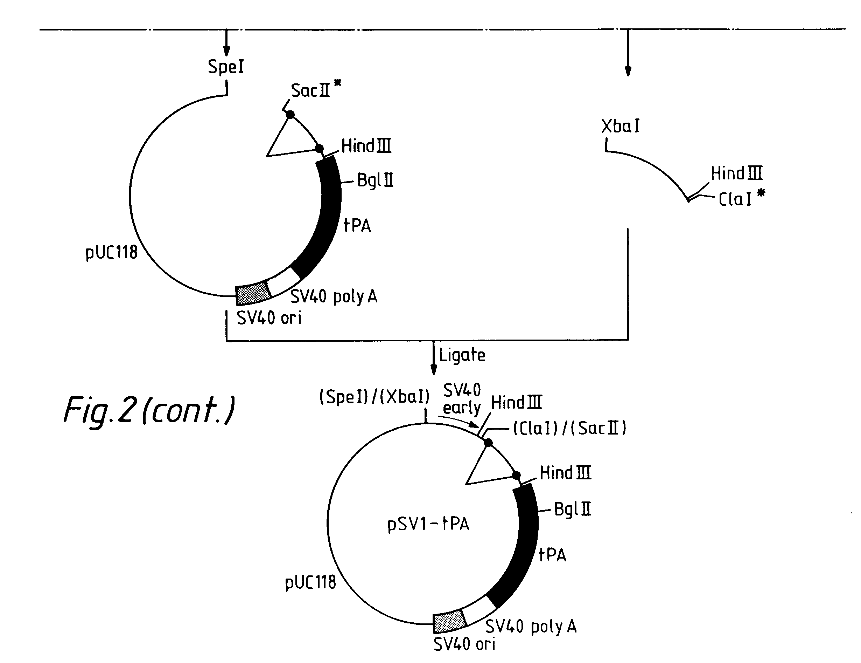

Mammalian expression vector pRK-tPA was prepared from pRK5 (described in EP 307,247, supra, where the pCIS2.8c28D starting plasmid is described in EP 278,776 published Aug. 17, 1988) and from t-PA cDNA (Pennica *et al*., Nature, 301: 214 [1983]). The cDNA was prepared for insertion into pRK5 by cutting with restriction endonuclease HindIII (which cuts 49 base pairs 5' of the ATG start codon) and restriction endonuclease BalI (which cuts 276 base pairs downstream of the TGA stop codon). This cDNA was ligated into pRK5 previously cut with HindIII and SmaI using standard ligation methodology (Maniatis *et al*., 1982, supra). This construct was named pRK-tPA, and is shown in Figure 2.

pRK-tPA drives the efficient synthesis of t-PA upon transient transfection into human 293 fibroblasts. The vector contains the cytomegalovirus immediate early gene enhancer and promoter, the CMV-IE splice donor site and a portion of the associated intron, the bacteriophage SP6 promoter, a portion of an IgV_{H} intron and the associated splice acceptor, the cDNA encoding t-PA, the SV40 early polyadenylation ("polyA") region, and the SV40 origin of replication ("ori") in plasmid pUC118.

The vector pE348DHFRUC (Vannice and Levinson, J. Virology, 62: 1305-1313 (1988), where it is designated pE, Figure 1) contains the SV40 enhancer and early promoter region upstream of the HindIII site (position 5171 in the virus), preceding cDNA encoding murine dihydrofolate reductase (DHFR), which is followed by the 584-bp Hepatitis B virus (HBV) polyA signal in plasmid pML1 from the BamHI to the BglII sites of HBV. This plasmid contains a polylinker immediately upstream of the SV40 sequences.

Portions of vectors pRK-tPA and pE348DHFRUC were isolated as follows (Fig. 2):
1. Vector pRK-tPA was digested with restriction enzyme SacII, and subsequently treated with the large ("Klenow") fragment of E. coli DNA polymerase I ("pol I") to remove the 3' protruding ends generated by SacII cleavage. This was followed by digestion with the restriction enzyme SpeI. The larger fragment containing a portion of the CMV transcribed sequences, the splice donor-intron-splice acceptor unit ("Intron" in Fig. 2), the t-PA cDNA, the SV40 polyA and ori regions, and pUC118, including a few nucleotides from the 5' end of CMV, was isolated from a polyacrylamide gel after electrophoretic separation of the fragments ("gel isolated").
2. Vector pE348DHFRUC was digested with the enzyme ClaI and the resulting 5' protruding ends were filled in using Klenow poll in the presence of all four deoxyribonucleotides (dNTPs: dATP, dGTP, dCTP, TTP). Upon subsequent digestion with XbaI, the SV40 transcription regulatory sequences (enhancer and early promoter, including the SV40 early sites of transcription initiation), present on the smaller XbaI-ClaI fragment (360 nt), were gel isolated.

The isolated pRK-tPA and pE348DHFRUC fragments were ligated to generate vector pSVI-tPA.

### b. pSVI2-tPA

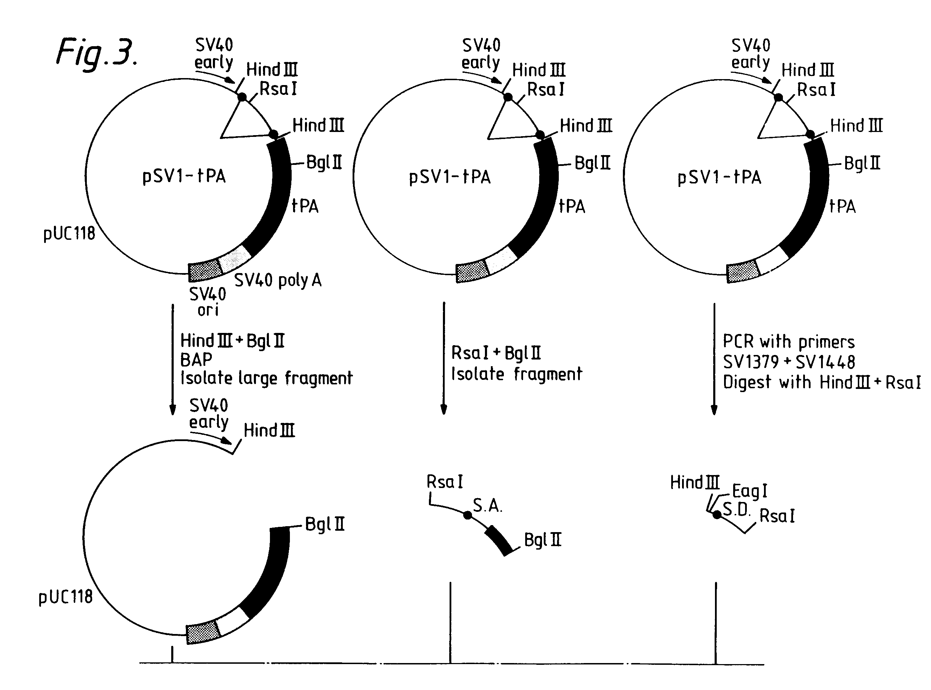

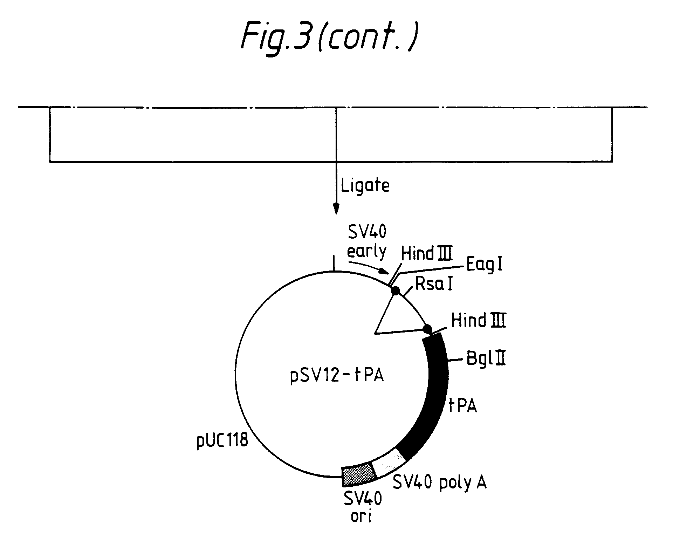

The vector pSVI2-tPA was prepared as shown in Figure 3, wherein the splice donor site of vector pSVI-tPA was mutated by ligation of three fragments, prepared as described below. Nucleotides -3 (G) and -1 (C) relative to the 5' exon/intron boundary of pSVI-tPA were changed to C and G, respectively, to render the splice donor sequence identical to the consensus sequence CAG/GUAAGU. This was accomplished as follows:
1. Vector pSVI-tPA was cleaved with HindIII and BglII to generate a small fragment containing primarily the splice donor-intron-splice acceptor unit between the two HindIII sites at positions 381 and 618, another small fragment containing a 5' portion of the t-PA cDNA located between the HindIII and BglII sites at positions 618 and 770, and a large fragment containing the remainder of the pSVI-tPA DNA. After bacterial alkaline phosphatase ("BAP") treatment, the three fragments were separated by gel electrophoresis and the largest was recovered.
2. Vector pSVI-tPA was separately digested with RsaI and BglII, and the 327-nt fragment extending from the RsaI site at position 443 to the BglII site at position 770 was gel isolated. This fragment includes the 3' portion of the intron, the splice acceptor, and a 5' portion of the t-PA cDNA.
3. Two oligonucleotides ("primers" in Figs. 3-7) were synthesized. The first (SVI379) corresponded to nucleotides 379 to 400 (upper strand) of pSVI-tPA, with the exception of a G to C change at position 390 introduced to generate the sequence recognized by restriction endonuclease EagI (CGGCCG), and overlapped a HindIII site. It had the following sequence (position of the change underlined):
   5'-AAAAGCTTATCCGGCCGGGAAC-3'.
   The second oligonucleotide (SVI448) was identical in sequence to the lower strand of pSVI-tPA between positions 448 and 427, except for a G to C change at the 11th nt and a C to G change at nt-13. It included a RsaI site just 5' to the two single nucleotide changes. The sequence of this oligonucleotide, with the differences from the corresponding pSVI-tPA sequenced underlined, was as follows:
   5'-CGGTACTTACCTGACTCTTGGC-3'.
   The two oligonucleotides were used to amplify by PCR the region of pSVI-tPA between positions 379 and 448, which includes the splice donor. The PCR products were digested with HindIII and RasI, and the 62-nt fragment was gel isolated.

The three isolated fragments were ligated and introduced into E. coli MM294. Plasmid DNA was isolated from several ampicillin-resistant colonies, and the nucleotide sequence of one isolate (pSVI2-tPA), that had the three fragments ligated in the desired order, was determined to verify the presence of the nucleotide changes specified by the two primers and the integrity of the region derived from the amplified fragment.

### c. pSVI3-tPA

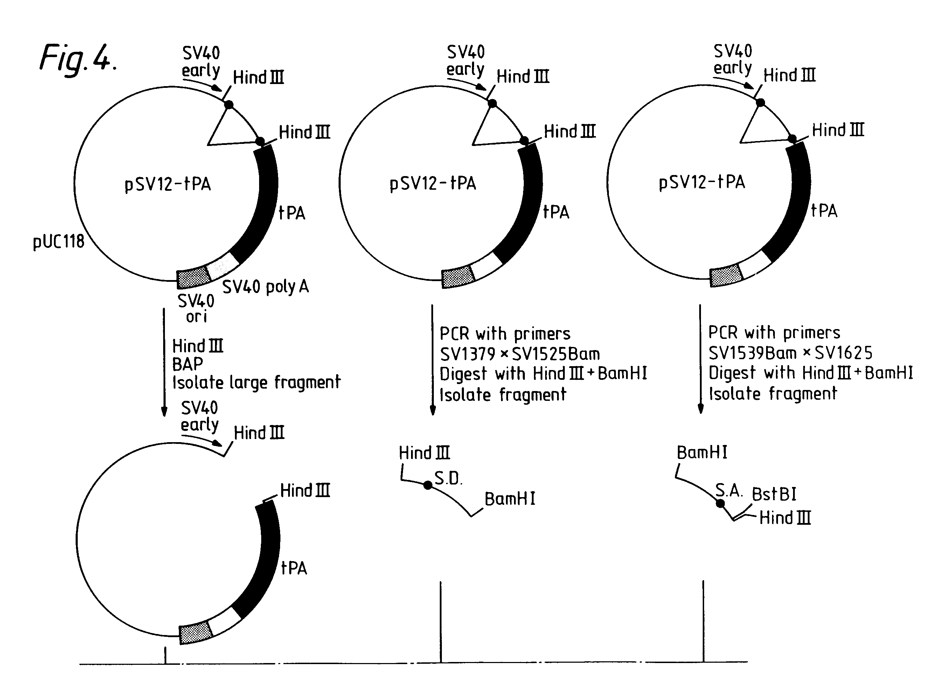

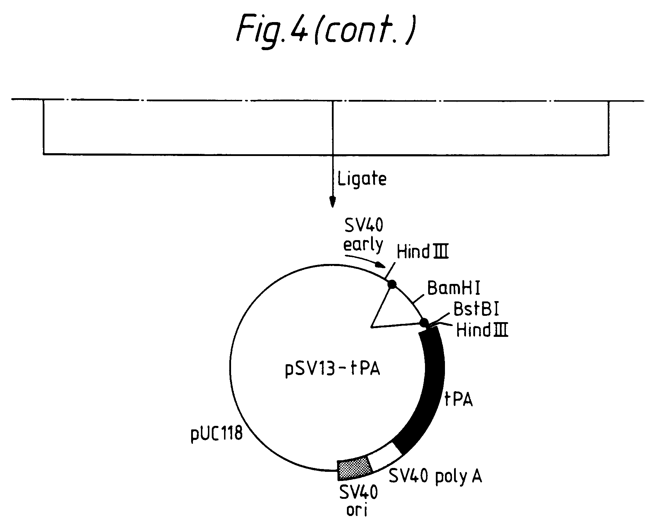

The vector pSVI3-tPA was prepared as shown in Figure 4. Two adjacent regions of pSVI2-tPA were amplified by PCR using in each case one oligonucleotide identical to the pSVI2-tPA sequence and one mutant oligonucleotide specifying the desired changes. These fragments were used to replace the corresponding regions in pSVI2-tPA. The fragments isolated were as follows:
1. Vector pSVI2-tPA was digested with HindIII, the digest treated with BAP, and the two HindIII fragments were separated by gel electrophoresis. The larger fragment was recovered from the gel; it contained all pSVI2-tPA sequences, except for the 237-nt HindIII fragment that includes the splice donor-intron-splice acceptor unit.
2. A new oligonucleotide (SVI525Bam) was synthesized for PCR mutagenesis of the ATG trinucleotide within the pSVI2-tPA intron. It was identical in sequence to the lower strand of pSVI2-tPA from nt 525 to nt 497, except for a T to A change at position 516, an A to C change at position 519, and a T to G change at position 523. The first change was designed to alter the ATG trinucleotide to TTG; the second and third to create the recognition sequence for the enzyme BamHI (GGATCC). The nucleotide sequence of SVI525Bam was as follows (differences from the pSVI2-tPA sequence underlined):
   5'-TAGGATCCAAAAGGTTATGTATTAATTGT-3'.
   Oligonucleotides SVI379 (see above) and SVI525Bam were used to amplify by PCR mutagenesis the region between positions 379 and 525 of pSVI2-tPA while incorporating the changes specified by SVI525Bam. The reaction products were digested with HindIII and BamHI and the resulting 137-nt fragment was gel isolated.
3. Oligonucleotide SVI539Bam was synthesized for PCR mutagenesis of the pSVI2-tPA branchpoint region. It corresponded to the pSVI2-tPA sequence (upper strand) from position 539 to position 573, but had the following changes: a G in place of the T at 541; a T in place of a C at 544; a C in place of an A at 545; a C in place of an A at 553; and a G in place of an A at 555. The first three changes created a BamHI recognition site, while the last two were intended to generate a signal resembling the BPS consensus. The nt sequence of SVI539Bam was as follows (changes from the pSVI2-tPA sequence underlined):
   5'-GGGGATCCTATAGACTGACATCCACTTTGCCTTTC-3'.
   Oligonucleotide SVI625 was synthesized and was identical to the lower strand of the pSVI2-tPA sequence from position 625 to position 603, except for an A to C change at position 617, designed to generate a BstBI restriction site (TTCGAA). The sequence of SVI625 was (change underlined):
   5'-CCAAGCTTCGAACCGAGGTGCAG-3'.
   Oligonucleotides SVI539Bam and SVI625 were used to amplify by PCR the region of pSVI2-tPA between nucleotides 539 and 625 and concurrently incorporate the desired changes. The PCR products were digested with HindIII and BamHI, and the 77-nt fragment was gel purified.

The three fragments (Fig. 4) were ligated and as such were introduced into E. coli MM294. Plasmid DNA was isolated from a number of ampicillin-resistant colonies and analyzed by digestion with appropriate restriction enzymes and gel electrophoresis for the presence of all three fragments in one copy each. The relative orientation of the fragments was also determined by this analysis. The nucleotide sequence of one recombinant plasmid (pSVI3-tPA), which had the three constituent fragments arranged in the same order as in pSVI2-tPA, was determined in the region spanning the three sites of recombination (the two HindIII sites and the BamHI site), including all sequences between these sites.

### d. pSVI5-tPA

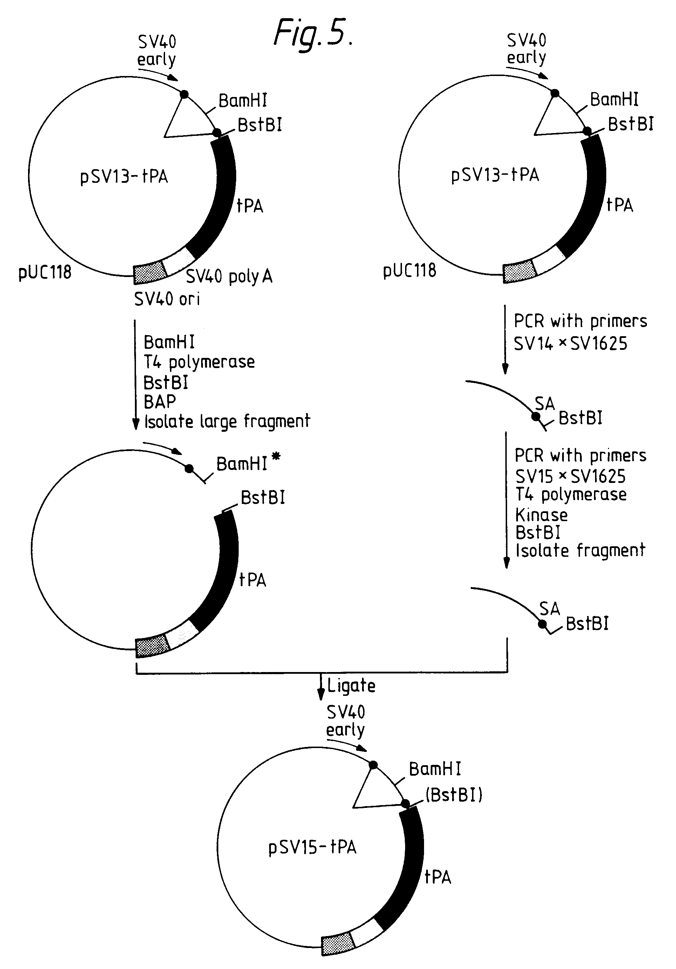

Vector pSVI5-tPA was constructed from pSVI3-tPA by replacing a portion of the intron and the splice acceptor with a PCR-generated mutant fragment (as shown in Figure 5). Nucleotide -16 (G) relative to the 3' splice junction of pSVI3-tPA was modified to the nucleotide T to create an uninterrupted 16-nt-long polypyrimidine tract as part of the splice acceptor. The vector also was modified to contain a potential branch acceptor nt at position -23 relative to the 3' splice junction, embedded within the sequence GACTTATT, which is complementary to the splice donor sequence (G/GTAAGT) in this vector. Overlapping this BPS is another BPS (TACTGAC) that conforms to the "wide" BPS consensus and is complementary to the U2 snRNA sequence. The branch acceptor nt in this BPS is located at position -27 relative to the 3' splice junction. Finally, the vector is modified by the insertion of a third BPS just upstream of the two BPSs in pSVI3-tPA. This third BPS is identical to the conserved yeast BPS (UACUAAC) and conforms to the "wide" mammalian BPS consensus (PyNCUPuAC). The branch acceptor in this BPS is located at position -34 relative to the 3' splice junction.

The fragments for generating pSVI5-tPA were prepared as follows:
1. Vector pSVI3-tPA was digested with BamHI and the resulting 5, protruding ends were filled in with T4 DNA polymerase in the presence of all four dNTPs. The material was subsequently digested with BstBI, treated with BAP, and the larger fragment containing all but part of the intron and the splice acceptor sequence of pSVI3-tPA gel isolated.
2. Two new oligonucleotides were synthesized for use in successive PCR amplifications without intermediate product isolation. This approach to introduce a variety of mutations was taken to avoid the use of a single long oligonucleotide with numerous mismatches to the target; longer synthetic oligonucleotides often contain a greater proportion of molecules of incorrect sequence. In addition, the first oligonucleotide alone could in the future be used to create a vector differing from pSVI3-tPA in the branchpoint and splice acceptor regions.

The first oligonucleotide, SVI4, had its 3' terminal thirteen nucleotides in common with pSVI3-tPA (positions 545-557). Preceding these were 22 nucleotides of sequence similar, but not identical, to the pSVI3-tPA sequence between positions 523 and 544. The differences were as follows (underlined below): a G to T change at position 528; an A to T at 535, a C to A at 537, a C to T at 538, and an A to T at 539; and a G to T change at position 544. The last change would cause an extension of the polypyrimidine tract of the splice acceptor; the first would alter the pSVI3-tPA sequence GACTGAC to conform to the BPS consensus sequence that matches U2. The remaining four single nucleotide changes were designed to create a sequence complementary to the splice donor. The sequence of SVI4 was:
5'-CTATATACTGACTTATTCTTTTCCTTTCTCTCCAC-3'.

The second oligonucleotide (SVI5) overlapped the central portion of SVI4. It differed in the five 5' terminal nt of SVI4 and extended in the 5' direction to incorporate the yeast BPS (TACTAAC). Its sequence was (underlined are the differences from the corresponding pSVI3-tPA sequence):
5'-CTACTAACTACTGACTTATTCTTT-3'.

The BPS/splice acceptor region of pSVI3-tPA was amplified and mutated by PCR mutagenesis using the oligonucleotide pair SVI4/SVI625. The PCR products were diluted and reamplified with oligonucleotide pair SVI5/SVI625. The products were treated with T4 DNA polymerase and T4 polynucleotide kinase and cleaved with restriction enzyme BstBI and the 71-nt fragment was gel isolated.

The two isolated fragments (Fig. 5) were ligated, and plasmid DNA was obtained from transformed (ampicillin-resistant) bacterial colonies and analyzed as described above for pSVI3-tPA. One isolate that carried all the desired intron changes was designated pSVI5-tPA.

Sequence determination identified an additional unforeseen change outside of the splice donor-intron-splice acceptor unit. Two additional nucleotides (GC) were present in pSVI5-tPA within the BstBI site of pSVI3-tPA (TTCGAA changed to TTCGCGAA), which site was therefore absent from pSVI5-tPA. The two additional nucleotides, however, created a recognition site for the enzyme NruI, which is unique in the vector. The additional nucleotides most likely resulted from an unanticipated filling-in of the BstBI protruding ends of the two constituent fragments used for ligation.

### e. pSVI6B-tPA

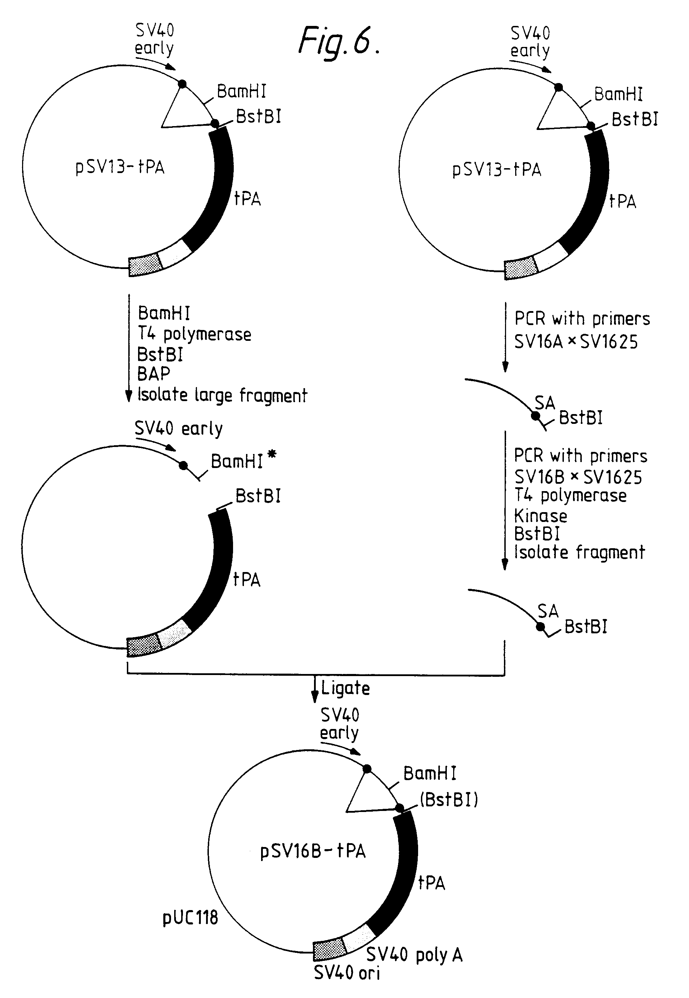

pSVI6B-tPA was created by an approach similar to the one described above for pSVI5-tPA, using two successive amplifications with partially overlapping mutant oligonucleotides to generate the desired changes in the intron-splice acceptor region of pSVI3-tPA (as shown in Fig. 6).

The first oligonucleotide (SVI6A) had the following sequence (differences from pSVI3-tPA, positions 523-550, underlined):
5'-CCTGACACTGACATCCACTTTTCCTTTC-3';
and the second (SVI6B; compare to positions 523-545 of pSVI3-tPA):
*5'-CTACTGACACTGACATCCACTTTTC-3'.*

Nucleotide replacements in SVI6A were: T to C at 524, T to G at 526, G to C at 528, and G to T at 544. The changes specified by SVI6B were: insertion of a C between nucleotides 525 and 526 of pSVI3-tPA, insertion of a G between nucleotides 526 and 527, replacement of the G at position 528 by a C, and the G to T change at position 544. These changes were designed to optimize the pSVI3-tPA BPS and introduce a second BPS just flanking it to the 5' side; these two branchpoint sequences overlap at the central C and are shown in italics in the SVI6B sequence.

Successive amplifications with oligonucleotides SVI6A and SVI6B in the presence of SVI625 were performed to modify the pSVI3-tPA intron-splice acceptor unit as described for pSVI5-tPA. Enzymatic treatment of the PCR products, gel isolation, and ligation to the pSVI3-tPA large BamHI-BstBI fragment were also as described for pSVI5-tPA. Nucleotide sequence analysis of plasmid DNA from one ampicillin-resistant bacterial colony (this plasmid isolate was designated pSVI6B-tPA) showed several unanticipated changes in addition to the modifications specified by the SVI6B oligonucleotide; position 545 was a T rather than the C of pSVI3-tPA; position 550 was also a T rather than a C; and the additional dinucleotide creating the NruI restriction site in pSVI5-tPA was also present in the pSVI6B-tPA sequence. The first two changes would not be expected to affect adversely t-PA expression and were not corrected.

### 2. Construction of Broad-Purpose Parental Vectors pSVI6B5 and pSVI6B7

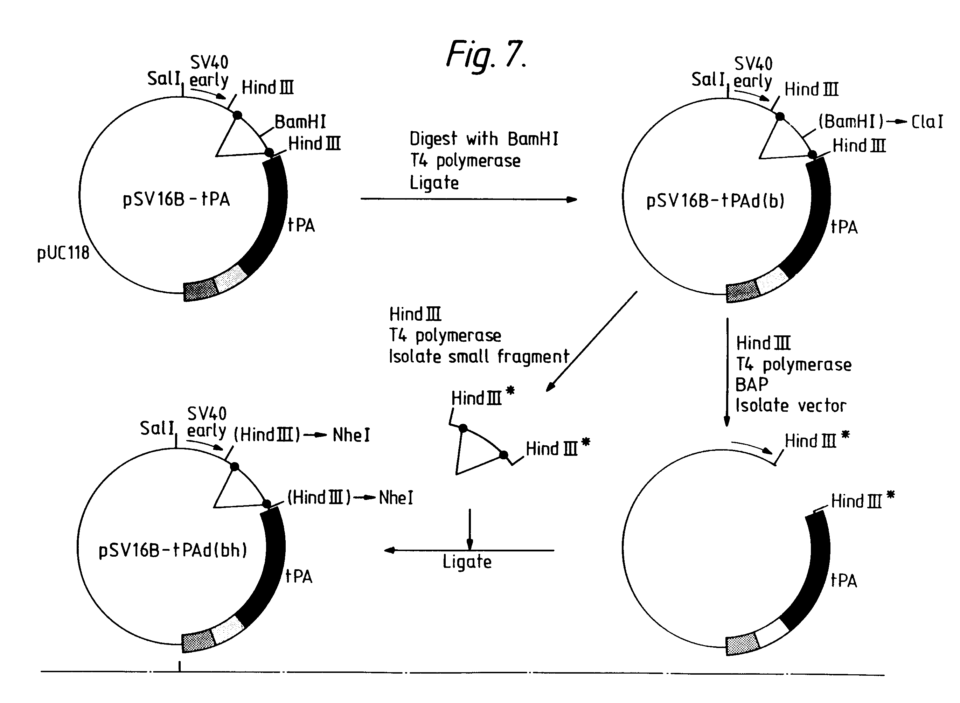

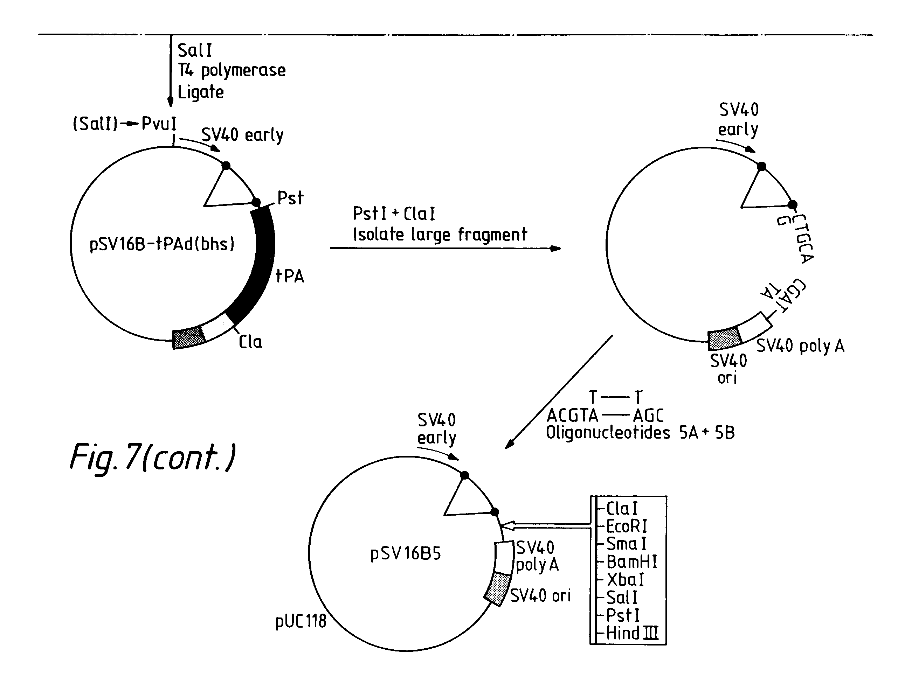

A broadly applicable parental vector for expression of different polypeptides was derived from pSVI6B-tPA, as shown in Figure 7. This vector, called pSVI6B5 (transformed E. coli strain ATCC No. 68,151) carries polylinker regions in place of the t-PA cDNA in pSVI6B-tPA. The polylinker for another useful parental vector, pSVI6B7 (from pRK7), is the same as that for pSVI6B5 (from pRK5, EP Pub. No. 307,247 published March 15, 1989), except that the order of the endonuclease restriction sites in the polylinker region between ClaI and HindIII is reversed. These polylinker regions provide convenient, unique restriction endonuclease recognition sites that can be used to introduce any sequence that encodes a polypeptide of interest, and expression of this sequence will be influenced by the modifications present in the pSVI6B-tPA splice unit.

Vector pSVI6B5 was generated in four steps, as described below and illustrated in Figure 7. The first three steps involved the removal of the BamHI, HindIII and SalI restriction sites, respectively, from pSVI6B-tPA; as a consequence, upon replacement of the t-PA cDNA by the polylinker in the last step, the polylinker sites for these enzymes were unique in the resulting parental expression plasmid.

### a. Construction of first intermediate plasmid bSVI6B-tPAd(b)

Vector pSVI6B-tPA was digested with BamHI, which cleaves only within the intron, the resulting 5' protruding ends were filled in with T4 DNA polymerase in the presence of dNTPs, and the linearized vector was gel isolated and treated with T4 DNA ligase to recircularize. This caused the loss of the BamHI recognition sequence; instead, the recognition sequence for the enzyme ClaI was created. However, due to methylation, ClaI is unable to cleave this sequence when plasmid DNA is obtained from dam⁺ E. coli.

### b. Construction of second intermediate plasmid DSVI6B-tPAd(bh)

Plasmid pSVI6B-tPAd(b) was digested with HindIII, which cleaves to both sides of the splice unit, the 5' protruding ends were filled in with T4 DNA polymerase in the presence of all four dNTPs, and part of the reaction mixture was treated with BAP. The two fragments present in both the treated and untreated material were separated by gel electrophoresis; the larger fragment was isolated from the treated material, and the smaller fragment from the untreated material. The two fragments were ligated to create plasmid pSVI6B-tPAd(bh). Filling in of the HindIII ends of both constituent fragments caused the loss of the two HindIII sites in the resulting plasmid and concurrently created two new recognition sites for the enzyme NheI.

### c. Construction of third intermediate plasmid pSVI6B-tPAd(bhs)

The single SalI recognition site was removed from plasmid pSVI6B-tPAd(bh) by digestion with this enzyme, T4 DNA polymerase treatment in the presence of dNTPs, gel isolation of the linear plasmid DNA, and recircularization using T4 DNA ligase. This resulted in the creation of a new PvuI recognition site.

### d. Construction of plasmids pSVI6B5 and pSVI6B7

For generation of the final, multi-purpose parental plasmid, two fragments were prepared as follows:
1 . Plasmid pSVI6B-tPAd(bhs) was digested with PstI and ClaI. There are three recognition sites for ClaI in this plasmid: one in the intron preceding the t-PA sequence, one at the boundary of the t-PA cDNA and the SV40 early polyA region, and one towards the end of the polyA region. Only one of these sites, the second, is insensitive to methylation. Consequently, plasmid DNA prepared from dam⁺ MM294 was cleaved by ClaI only at this site. A PstI recognition site is located at the junction of the splice unit and the t-PA cDNA, and several more are present in the t-PA sequence between this position and the methylation-insensitive ClaI site. Cleavage with PstI and ClaI thus resulted in the release of several smaller fragments containing t-PA sequences, and a large fragment containing all pSVI6B-tPAd(bhs) sequences with the exception of the t-PA cDNA. This larger fragment was gel isolated.
2. Two oligonucleotides were synthesized. These were complementary over the entire length (47 nt) of the first (designated 5A). The second, 5B, was extended at its 5' end by two nucleotides, and at its 3' end by four (underlined in the 5B sequence below). Annealing of these complementary oligonucleotides thus resulted in a DNA fragment having one 5' and one 3' protruding end ("overhang"). The sequence of the four-nt 3' protruding end was TGCA-3', which is complementary to the 3' overhang created by PstI cleavage of PstI recognition sites. The two-nt 5' protruding end consisted of the dinucleotide 5'-CG, which is complementary to the 5' overhang created by ClaI cleavage of ClaI restriction sites. The sequences of the two oligonucleotides were further designed to provide recognition sites for a number of restriction endonucleases (some of these are indicated under the sequence of each oligonucleotide shown below). The sequence of oligonucleotide 5A was: 
5'-TCGATTGAATTCCCCGGGGATCCTCTAGAGTCGACCTGCAGAAGCTT-3'
EcoRI SmaI BamHI XbaI SalI PstI HindIII
 The sequence of oligonucleotide 5B was: 
5'-CGAAGCTTCTGCAGGTCGACTCTAGAGGATCCCCGGGGAATTCAATCGATGCA-3'
HindIII PstI SalI XbaI BamHI SmaI EcoRI ClaI
 Oligonucleotides 5A and 5B were annealed and ligated to the isolated pSVI6B-tPAd(bhs) fragment to create plasmid pSVI6B5. Ligation of the PstI overhang to the TGCA-3' overhang of the annealed oligonucleotides did not regenerate a PstI site, but created a ClaI site; at the other end of the linker, ligation of the ClaI overhang to the 5'-CG overhang did not regenerate a ClaI site.

Plasmid pSVI6B7 was created in a similar manner, using the following two oligonucleotides (7A and 7B; note that the order of the restriction sites in these oligonucleotides is reversed relative to the protruding ends when compared with oligonucleotides 5A and 5B):

7A
5'-AAGCTTCTGCAGGTCGACTCTAGAGGATCCCCGGGAATTCAAT-3'
HindIII PstI SalI XbaI BamHI SmaI EcoRI
7B
5'-CGATTGAATTCCCCGGGATCCTCTAGAGTCGACCTGCAGAAGCTTTGCA-3'
EcoRI SmaI BamHI XbaI SalI PstI HindIII

### 3. Construction of pSVI6B-LAP-ARG and pSVI6B-LAP-SER and Expression of Gene Encoding Latency Associated Peptide

### a. Preparation of Plasmids

The polypeptide produced in this example is the latency associated peptide (LAP) that remains when mature TGF-β is cleaved from its precursor remainder. This peptide can be used to scavenge excess TGF-β or keep TGF-β latent until it reaches the site for activation.

Figure 8 depicts the complete nucleotide and predicted amino acid sequence of LAP. Predicted amino acids of the peptide are shown below the DNA sequence and are numbered from the first residue of the N-terminus of the peptide sequence. The mature TGF-β sequence is not included. Negative numbers indicate the signal sequence, and positive numbers designate the sequence of the secreted peptide.

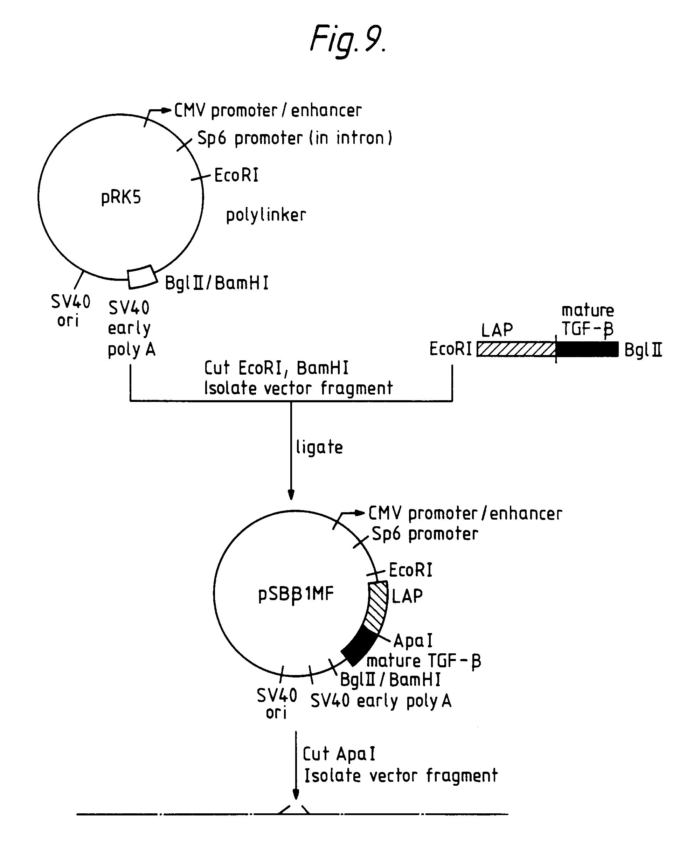

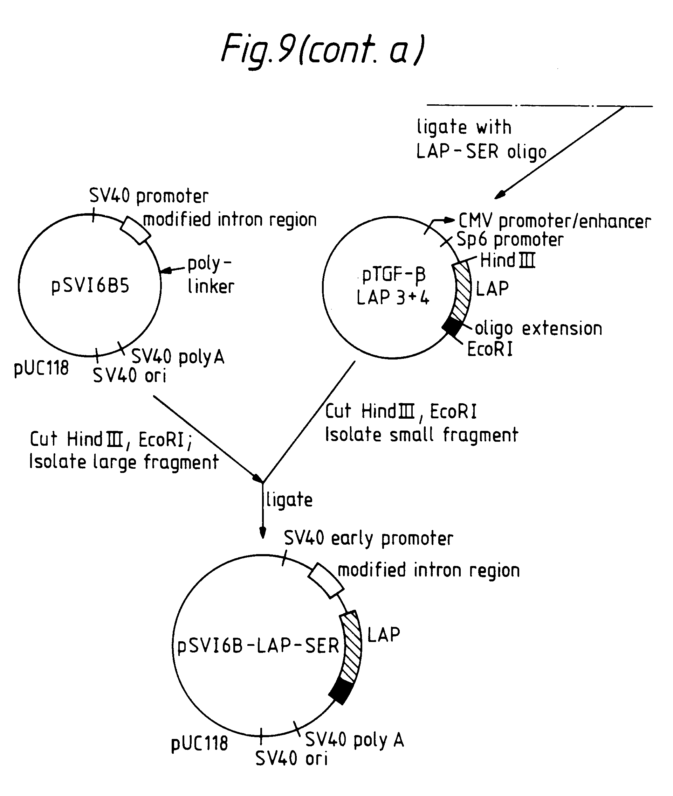

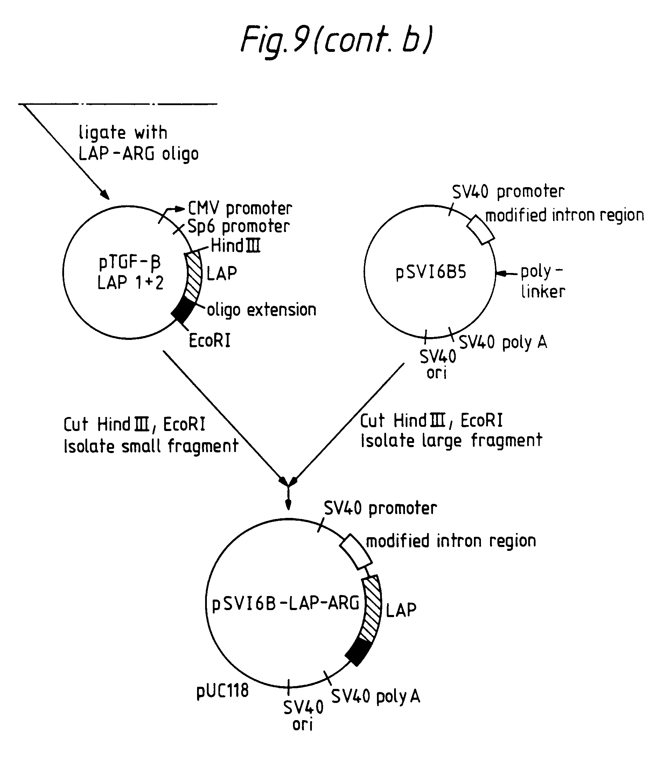

Plasmid pSVI6B-LAP-SER contains the sequences for the pre-pro region of TGF-β1 terminating at serine residue #275, which normally precedes the basic cleavage sites in the TGF-β1 precursor. The plasmid pSVI6B-LAP-ARG is the same except that it terminates at arginine residue #279, which is encoded by a codon just 5' of a stop codon. The construction of pSVI6B-LAP-SER and pSVI6B-LAP-ARG is shown in Figure 9. The plasmid pSVI6B-LAP-ARG was transformed into an E. coli strain, and the resulting host was deposited in the American Type Culture Collection, Rockville, MD on October 25, 1989, as ATCC No. 68,150.

In detail, these vectors were prepared as follows: Open reading frame DNA encoding preproTGF-β terminated with EcoRI and BglII (see EP Pub. 200,341 published Dec. 10, 1986) was isolated. The vector pRK5 was cleaved with EcoRI and BamHI and the large vector fragment was isolated. The small EcoRI-BglII fragment and the large pRK5 vector fragment were ligated to result in plasmid pSBβIMF, which has a 21-bp deletion of the polylinker sequence of pRK5. pSBβIMF has the precursor TGF-β-encoding DNA driven by the CMV promoter/enhancer followed by a splice-donor-intron-splice-acceptor region 5' to the TGF-β-encoding DNA. At the 3' end of the TGF-β-encoding DNA is SV40 early polyA and an SV40 origin of replication.

The vector pSBβIMF was digested with ApaI and HindIII and the large vector fragment was isolated. This fragment was ligated with one of the two oligonucleotides below: 
LAP-ARG: ApaI 5'-CAGCATCTGCAAAGCTCCCGGCACCGCCGATA(A)   HindIII
              CGGGTCGTAGACGTTTCGAGGGCCGTGGCGGCTAT T TCGA
                 GlnHisLeuGlnSerSerArgHisArgArgStop
or
LAP-SER: ApaI 5'-CAGCATCTGCAAAGCTCCTA(A)   HindIII
              CGGGTCGTAGACGTTTCGAGGAT T TCGA
                 GlnHisLeuGlnSerSerStop
The resulting plasmids, pTGF-βLAP1+2 and pTGF-βLAP3+4, were cleaved with EcoRI and HindIII and the small fragments were isolated. These fragments were then ligated into the isolated vector fragment from pSVI6B5 that had been cut with EcoRI and HindIII so that the LAP fragments were inserted into the polylinker region of the vector. The resulting plasmids were named pSVI6B-LAP-SER and pSVI6B-LAP-ARG.

### b. Transient Expression

Human 293S cells (Graham and van der Eb, Virology, 52: 456-467 [1973]) seeded at about 30% confluency in 60-mm dishes were transfected (Gorman *et al*., Science, 221: 551-553 [1983]) the following day with 2.5 »g of pSVI6B-LAP-SER or pSVI6B-LAP-ARG and 2 »g of plasmid RSV T-antigen. The next day cells were labeled in 1.0 ml serum-free DMEM with 250 »Ci/ml each of ³⁵S-cysteine and ³⁵S-methionine for 1-2 hours. Labeling medium was removed and secreted proteins were collected for 4-5 hours in 1 ml of serum-free medium. The supernatant was cleared by centrifugation and rinsed cells were lysed in 0.5 ml lysis buffer (1% Nonidet P40; 0.5% deoxycholate; 0.1% SDS; 5 mM EDTA in phosphate-buffered saline) and spun for ten minutes and the lysate was frozen until use.

Secreted protein (100 »l) were immunoprecipitated with a murine antibody raised against recombinant human TGF-β1. Supernatants to be precipitated with the TGF-β antibody were heated at 75°C for 5 minutes in the presence of 0.1% BSA and chilled on ice before addition of antibody. Immune complexes of secreted proteins were precipitated with Pansorbin (Calbiochem) and cell lysates with Protein A-Sepharose (Pharmacia). Denatured immunoprecipitates or total secreted proteins (15 »l) were electrophoresed on 13% SDS-PAGE, gels were permeated with Enhance (DuPont) and exposed three days at -70°C.

An autoradiograph of an SDS-PAGE of ³⁵S-labeled total secreted proteins from the transfected cells indicates that the pSVI6B-LAP-SER yields a protein at about 75 Kd, representing the LAP. An autoradiograph of SDS-PAGE analysis of TGF-β1 immuno-representing the LAP. An autoradiograph of SDS-PAGE analysis of TGF-β1 immuno-precipitation indicated that LAP did not immunoprecipitate with the antibody directed against mature TGF-β1.

### c. Stable Expression

CHO-dhfr⁻ cells (such as those described by Urlaub and Chasin, supra) were cotransfected with either pSVI6B-LAP-SER or pSVI6B-LAP-ARG and with a dhfr selection vector pFD11 (Simonsen and Levinson, Proc. Natl. Acad. Sci. USA, 80: 2495-2499 [1983]) using the general procedure of Graham and van der Eb, supra. The latter plasmid encodes DHFR, thereby conferring methotrexate resistance on the transfected cells and allowing for selection of LAP-expressing transformants. The transformed dhfr- cells were selected by growth in glycine, hypoxanthine, and thymidine deficient medium. Colonies that arose on this selection medium were isolated using cotton swabs and propagated in the same medium to several generations. After cell growth the cells were amplified and selected with increasing amounts of methotrexate using standard techniques.

The cells were then labeled and analyzed as discussed above for transient expression. Both transfections yielded a protein at about 75 Kd, representing the LAP-SER or LAP-ARG segment.

### EXAMPLE 2

### Bioactivity of LAP Secreted from 2935 and CHO Cells

### 1. Transient S293 Cell Transfection Bioactivity

Serum-free conditioned medium was collected from 293S cells transfected with either pSVI6B-LAP-SER or pRKTGF-β1, which is pRK5 containing full-length TGF-β (precursor TGF-β) as the insert. This conditioned media was used to examine the effects of the LAP on TGF-β in the mink lung fibroblast assay used as a standard bioassay for TGF-β. The TGF-β supernatant was heat activated by treatment at 75°C for 5 minutes, and a series of dilutions from 1:100, 1:200, 1:400, 1:800, and 1:1600 were prepared. A 1:200 dilution of the LAP supernatant, which was either untreated or heat treated, was added to each of these dilutions before submission to the assay.

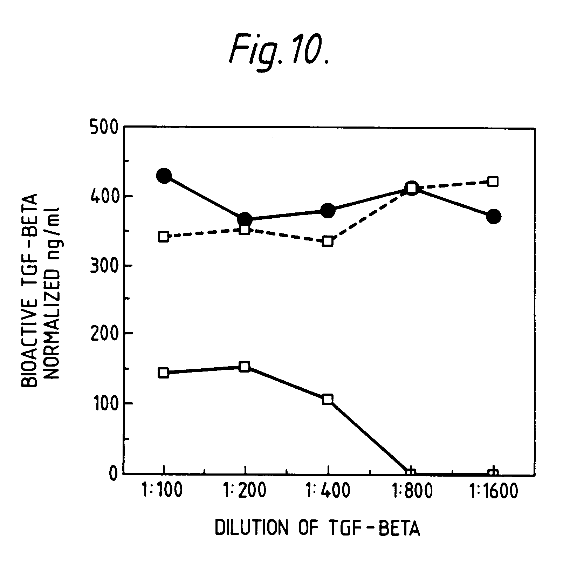

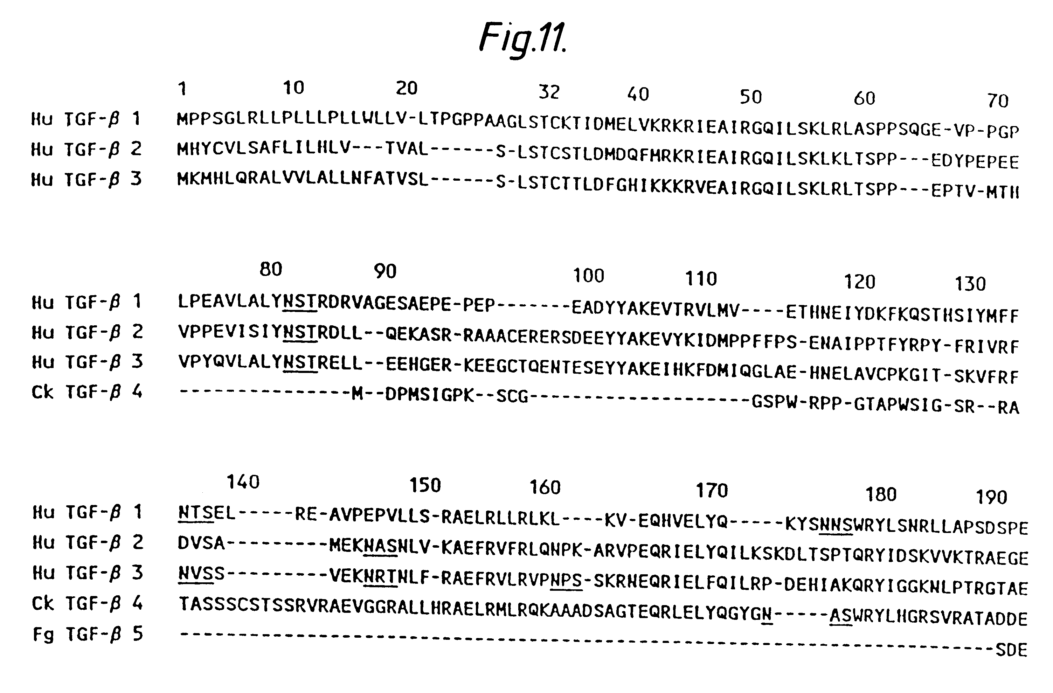

As shown in Fig. 10, where the circles are heat-activated TGF-β, the squares with dashed lines are heat-inactivated LAP, and the squares with solid lines are untreated LAP, the addition of heat-treated LAP did not affect the biological activity of TGF-β, indicating that the heat irreversibly inactivated the LAP. In contrast, the presence of untreated LAP protein was able either to reduce the amount of TGF-β activity (1:100 to 1:400 dilutions) or to negate totally the TGF-β activity (1:800 to 1:1600) when present in excess.

Addition to a mink lung fibroblast cell line of untreated LAP supernatant reduced the baseline production of TGF-β1 from 3 ng to 0 ng of TGF-β1. This result showed that the LAP had reversed the inhibition activity of the TGF-β.

### 2. Stable CHO Cell Transfection Bioactivity

Serum-free conditioned medium (2.5 ml) was collected from the cells selected using methotrexate as described above in Example 1c. The medium was evaluated for bioactivity as described above for the transfected S293 cells, and the results are shown in the table below.

| Amount of Conditioned Media | Bioassay Activity of TGF-β (ng/ml) | |
|---|---|---|
| | pSVI6B-LAP-ARG | pSVI6B-LAP-SER |
| 0 | 2726 | 2762 |
| 10 lambda | 1795 | 907 |
| 50 lambda | 627 | LTS |
| 250 lambda | 171 | LTS |
| 250 lambda, heat | 2698 | 2882 |

The difference in activities between the LAPs generated by the two plasmids (ability to inactivate mature TGF-β) relates to the amount of each cell line required to get the protein out of the cell and is not due to the specific activity of the form of LAP generated. The results indicate that as the concentration of untreated LAP supernatant increased, the TGF-β activity decreased in a linear fashion.

### EXAMPLE 3

### Assay for TNF-α

### 1. Cytotoxic Assay Procedure

The L-929 assay system is a convenient *in vitro* assay that permits rapid measurement of the activity of the LAP herein optionally in conjunction with an appropriate regulator of immune function. Its degree of correlation with the *in vivo* tumor necrosis assay of Carswell *et al.*, Proc. Natl. Acad. Sci. USA, 72:3666 (1975) is at present unknown; however, as it utilizes murine tumor cells specifically, the correlation is expected to be high. The proteins tumor necrosis factor (TNF-α) and lymphotoxin (TNF-β) give activity in this assay. The assay is similar in concept to that disclosed in U.S. Pat. No. 4,457,916, which used murine L-M cells and methylene blue staining. However, the L-929 assay has been shown to correlate (for TNF-α derived from HL-60 cells) with human tumor cell line cytotoxicity.

In the L-929 assay system herein, L-929 cells are prepared overnight as monolayers in microtiter plates. The best samples are diluted twofold across the plate, UV irradiated, and then added onto the prepared cell monolayers. The culture media in the wells are then brought to 1 »g/ml actinomycin D. The plates are allowed to incubate 18 hours at 37°C and the plates are scored visually under the microscope. Each well is given a 25, 50, 75, or 100% mark signifying the extent of cell death in the well. One unit of TNF activity is defined as the reciprocal of the dilution at which 50% killing occurs.

### 2. In Vivo Assays

Preparations may also be tested for activity using the ability of the drug to kill or repress the growth of tumors and to protect the animal bearing the tumor from mortality. Balb/c mice are injected subcutaneously with various types of tumor cells to create a localized tumor. Tumor cell lines include MethA mouse fibrosarcoma, obtained as a cell suspension from ascites fluid, and MCF-7, a human breast carcinoma that is administered as a 1 mm³ clump of cells.

For the assay, female Balb/c mice (19-22 g) are injected subcutaneously by 26-gauge needle with either suspension containing 5 x 10⁵ fibrosarcoma cells in 0.1 ml medium or with the MCF-7 clumps. (The fibrosarcoma suspension is prepared from 8-day-old ascites fluid by cell counting and dilution with serum-free medium.) After 9-10 days, when the tumor becomes palpable, 1 »g per mouse of TNF-α is injected intravenously, and administration of the TNF-α is repeated, if desired, on subsequent days. Results are assessed by measuring tumor volume and by survival rate. The test is repeated using separate sequential injections of 1 »g per mouse of TNF-α and 10 mg/kg per mouse of LAP prepared as described in Example 1 and purified by standard methods.

The LAP is found to enhance the tumor-reduction activity of the TNF-α in both the *in vivo* and *in vitro* tests described above.

### EXAMPLE 4

### Assay for M-CSF

In an assay for determining sarcoma cell killing, normal 2-hour adherent C3H/HeN mouse peritoneal macrophages are incubated for one day *in vitro* with and without murine rM-CSF as murine L-cell-conditioned medium containing 1200 U/ml M-CSF (prepared as in U.S. Pat. No. 4,847,201) and with a formulation containing the above murine M-CSF medium and 1 mg/ml of the LAP used in Example 3. Then the incubates are mixed at a 20:1 ratio with ³H-thymidine-labeled mouse sarcoma TU5 cells along with 10% v/v conA-induced (10 »g/ml) spleen lymphokine (LK), which contains gamma-interferon. The release of labeled thymidine over the following 48-hour period is used as a measure of tumor cell killing.

The LAP is found to increase the ability of the M-CSF to stimulate tumor cell killing by murine macrophages.

### EXAMPLE 5

### Assay for IL-2

The LAP used in Example 3, when added to purified native IL-2 isolated from the induced Jurkat cell line, is found to increase the effectiveness of the IL-2, as measured by its specific bioactivity in the HT-2 cell proliferation assay (Watson, J.Exp. Med, 150: 1510-1519 (1979) and Gillis *et al*., J. Immunol., 1230: 2027-2032 [1979]).

### EXAMPLE 6

A 60-year-old male patient weighing 90 kg, having a deep wound to his leg and suffering from a suppressed immune system leading to soft tissue infection, is injected with one bolus injection of 10 mg/kg of the LAP used in Example 3 formulated with 5 mg human serum albumin, 8 mg sodium chloride, 0.2 mg potassium hydrogen phosphate, and 1.4 mg disodium hydrogen phosphate. After two weeks of therapy, the infection of the patient is resolved.

### EXAMPLE 7

A 55-year-old male patient weighing 70 kg suffering from a Streptococcal infection is immunosuppressed and therefore contracts a secondary infection. He is continuously infused with a composition having a dose of 0.1 mg/kg of the LAP used in Example 3 for five weeks. As a result of treatment, the secondary infection is resolved.

### EXAMPLE 8

A 50-year-old male patient weighing 85 kg having a deep incision on his arm as a result of surgery and suffering from soft tissue infection resulting from his immunosuppressed condition is injected intravenously with a formulation containing a dose of 25 »g/m² recombinant human IFN-gamma (U.S. Pat. No. 4,762,791), 1 mg/kg of the LAP used in Example 3, 5 mg human serum albumin, 8 mg sodium chloride, 0.2 mg potassium hydrogen phosphate, and 1.4 mg disodium hydrogen phosphate. This treatment is carried out five times a week for four weeks. At the end of four weeks the infection is resolved.

### EXAMPLE 9

A male patient 35 years old and weighing 75 kg suffers with a third degree burn on 50% of his body and a suppressed immune system leading to soft tissue infection. He is injected intravenously with the composition of Example 6 for 3 to 5 times per week for 5 weeks. After 3 weeks of therapy, the patient's superficial infection resolves.

### Deposit of Materials

The following strains have been deposited with the American Type Culture Collection, 12301 Parklawn Drive, Rockville, MD, USA (ATCC):

| Strains | ATCC Accession No. | Deposit Date |
|---|---|---|
| 294.pSVI6B-LAP-ARG | 68,150 | October 25, 1989 |
| 294/pSVI6B5 | 68,151 | October 25, 1989 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture for 30 years from the date of deposit. The organisms will be made available by ATCC under the terms of the Budapest Treaty, and subject to an agreement between Genentech, Inc. and ATCC, which assures permanent and unrestricted availability of the progeny of the cultures to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC §122 and the Commissioner's rules pursuant thereto (including 37 CFR §1.14 with particular reference to 886 OG 638).

The assignee of the present application has agreed that if the cultures on deposit should die or be lost or destroyed when cultivated under suitable conditions, they will be promptly replaced on notification with a viable specimen of the same culture. Availability of the deposited cultures is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the cultures deposited, since the deposited embodiments are intended as separate illustrations of certain aspects of the invention and any cultures that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

## Claims (Claims for the following Contracting State(s): AT, BE, CH, DE, DK, FR, GB, IT, LI, LU, NL, SE)

1. An isolated nucleic acid sequence comprising a DNA sequence encoding the peptide shown in Figure 8, or analogues or variants thereof, having the biological activity of a latency associated peptide, provided that the sequence does not also encode a mature TGF-β.

2. A nucleic acid sequence of claim 1 wherein the peptide encoded by the nucleic acid sequence has at least an 80% amino acid sequence identity with the recombinant latency associated peptide of Figure 8.

3. An expression vector comprising the nucleic acid sequence of claim 1 or claim 2 operably linked to control sequences recognized by a host transformed by the vector.

4. The vector of claim 3 that is pSV16B-LAP-SER as shown in Figure 9 (cont. a).

5. The vector of claim 3 that is pSV16B-LAP-ARG deposited under ATCC 68150.

6. A host cell transformed with the expression vector of any one of claims 3 to 5.

7. A method of producing a latency associated peptide, which method comprises culturing the cells of claim 6 to express nucleic acid encoding the peptide in the host cell culture.

8. A latency associated peptide as obtainable by the method of claim 7.

9. Latency associated peptide having a molecular weight of about 75,000 when measured by non-reducing SDS-PAGE and capable of antagonizing a biological activity of mature TGF-β, which peptide is completely free of source proteins.

10. A method for preparing a pharmaceutical composition useful for antagonizing a TGF-β activity, which method comprises mixing a therapeutically effective amount of the peptide of claim 8 or claim 9 with a pharmaceutically acceptable carrier.

11. A method for preparing a pharmaceutical composition useful as having a TGF-β activity, which method comprises mixing a therapeutically effective amount of the peptide of claim 8 or claim 9 with a pharmaceutically acceptable carrier.

12. A method for diagnosing the presence of a disease or disorder detectable by the presence of mature TGF-β in a serum sample of a patient comprising adding to the serum sample a labelled form of the peptide of claim 8 or claim 9 and assaying for the presence of labelled complexes of the peptide and mature TGF-β in the serum sample.

13. The peptide of claim 8 or claim 9 for use in a method of medical treatment or diagnosis.

14. Use of the peptide of claim 8 or claim 9 in the preparation of a medicament for antagonizing a TGF-β activity in a mammal.

15. Use of the peptide of claim 8 or claim 9 and TGF-β in the preparation of a medicament for treating a mammal in need of TGF-β treatment.

## Claims (Claims for the following Contracting State(s): ES)

1. A process which comprises producing an isolated nucleic acid sequence comprising a DNA sequence encoding the peptide shown in Figure 8, or analogues or variants thereof, having the biological activity of a latency associated peptide, provided that the sequence does not also encode a mature TGF-β.

2. The process of claim 1 wherein the peptide encoded by the nucleic acid sequence has at least an 80% amino acid sequence identity with the recombinant latency associated peptide of Figure 8.

3. A process which comprises producing an expression vector comprising the nucleic acid sequence of claim 1 or claim 2 operably linked to control sequences recognized by a host transformed by the vector.

4. The process of claim 3 wherein the vector is pSV16B-LAP-SER as shown in Figure 9 (cont. a).

5. The process of claim 3 wherein the vector is pSV16B-LAP-ARG deposited under ATCC 68150.

6. A host cell transformed with the expression vector of any one of claims 3 to 5.

7. A method of producing a latency associated peptide, which method comprises culturing the cells of claim 6 to express nucleic acid encoding the peptide in the host cell culture.

8. A process which comprises producing latency associated peptide having a molecular weight of about 75,000 when measured by non-reducing SDS-PAGE and capable of antagonizing a biological activity of mature TGF-β, which peptide is completely free of source proteins.

9. A method for preparing a pharmaceutical composition useful for antagonizing a TGF-β activity, which method comprises mixing a therapeutically effective amount of the peptide of claim 8 with a pharmaceutically acceptable carrier.

10. A method for preparing a pharmaceutical composition useful as having a TGF-β activity, which method comprises mixing a therapeutically effective amount of the peptide of claim 8 with a pharmaceutically acceptable carrier.

11. A method for diagnosing the presence of a disease or disorder detectable by the presence of mature TGF-β in a serum sample of a patient comprising adding to the serum sample a labelled form of the peptide of claim 8 and assaying for the presence of labelled complexes of the peptide and mature TGF-β in the serum sample.

12. Use of the peptide of claim 8 in the preparation of a medicament for antagonizing a TGF-β activity in a mammal.

13. Use of the peptide of claim 8 and TGF-β in the preparation of a medicament for treating a mammal in need of TGF-β treatment.

## Claims (Claims for the following Contracting State(s): GR)

1. An isolated nucleic acid sequence comprising a DNA sequence encoding the peptide shown in Figure 8, or analogues or variants thereof, having the biological activity of a latency associated peptide, provided that the sequence does not also encode a mature TGF-β.

2. A nucleic acid sequence of claim 1 wherein the peptide encoded by the nucleic acid sequence has at least an 80% amino acid sequence identity with the recombinant latency associated peptide of Figure 8.

3. An expression vector comprising the nucleic acid sequence of claim 1 or claim 2 operably linked to control sequences recognized by a host transformed by the vector.

4. The vector of claim 3 that is pSV16B-LAP-SER as shown in Figure 9 (cont. a).

5. The vector of claim 3 that is pSV16B-LAP-ARG deposited under ATCC 68150.

6. A host cell transformed with the expression vector of any one of claims 3 to 5.

7. A method of producing a latency associated peptide, which method comprises culturing the cells of claim 6 to express nucleic acid encoding the peptide in the host cell culture.

8. A latency associated peptide as obtainable by the method of claim 7.

9. Latency associated peptide having a molecular weight of about 75,000 when measured by non-reducing SDS-PAGE and capable of antagonizing a biological activity of mature TGF-β, which peptide is completely free of source proteins.

10. A pharmaceutical composition useful for antagonizing a TGF-β activity comprising a therapeutically effective amount of the peptide of claim 8 or claim 9 in a pharmaceutically acceptable carrier.

11. A pharmaceutical composition useful as having a TGF-β activity comprising a therapeutically effective amount of the peptide of claim 8 or claim 9 and a therapeutically effective amount of a mature TGF-β in a pharmaceutically acceptable carrier.

12. A method for diagnosing the presence of a disease or disorder detectable by the presence of mature TGF-β in a serum sample of a patient comprising adding to the serum sample a labelled form of the peptide of claim 8 or claim 9 and assaying for the presence of labelled complexes of the peptide and mature TGF-β in the serum sample.

13. The peptide of claim 8 or claim 9 for use in a method of medical treatment or diagnosis.

14. Use of the peptide of claim 8 or claim 9 in the preparation of a medicament for antagonizing a TGF-β activity in a mammal.

15. Use of the peptide of claim 8 or claim 9 and TGF-β in the preparation of a medicament for treating a mammal in need of TGF-β treatment.

## Patentansprüche (Patentansprüche für folgende(n) Vertragsstaat(en): AT, BE, CH, DE, DK, FR, GB, IT, LI, LU, NL, SE)

1. Isolierte Nukleinsäure-Sequenz, umfassend eine für das in Fig. 8 dargestellte Peptid kodierende DNA-Sequenz oder Analoge oder Varianten davon, die die biologische Aktivität eines latenzassoziierten Peptids besitzt, vorausgesetzt, daß die Sequenz nicht auch für einen reifen TGF-β kodiert.

2. Nukleinsäure-Sequenz nach Anspruch 1, worin das von der Nukleinsäure-Sequenz kodierte Peptid eine zumindest 80%-ige Aminosäuresequenz-Identität mit dem rekombinanten, latenzassoziierten Peptid aus Fig. 8 aufweist.

3. Expressionsvektor, umfassend die Nukleinsäure-Sequenz nach Anspruch 1 oder 2, die mit Steuersequenzen operabel verbunden ist, die von einem mit dem Vektor transformierten Wirt erkannt werden.

4. Vektor nach Anspruch 3, der der in Fig. 9 (Forts. a) dargestellte Vektor pSV16B-LAP-SER ist.

5. Vektor nach Anspruch 3, der der unter ATCC 68.150 hinterlegte Vektor pSV16B-LAP-ARG ist.

6. Mit dem Expressionsvektor nach irgendeinem der Ansprüche 3 - 5 transformierte Wirtszelle.

7. Verfahren zur Herstellung eines latenzassoziierten Peptids, umfassend das Kultivieren der Zellen nach Anspruch 6 zum Exprimieren von Nukleinsäure, die für das Peptid in der Wirtszellenkultur kodiert.

8. Latenzassoziiertes Peptid, das nach dem Verfahren nach Anspruch 7 erhalten werden kann.

9. Latenzassoziiertes Peptid mit einem Molekulargewicht von etwa 75.000, gemessen durch nicht-reduzierende SDS-PAGE, das in der Lage ist, einer biologischen Aktivität von reifem TGF-β entgegenzuwirken, undvollständig frei von Ursprungsproteinen ist.

10. Pharmazeutische Zusammensetzung, die zum Entgegenwirken gegen eine TGF-β-Aktivität dient, umfassend eine therapeutisch wirksame Menge des Peptids nach Anspruch 8 oder 9 in einem pharmazeutisch annehmbaren Träger.

11. Pharmazeutische Zusammensetzung, die aufgrund einer TGF-β-Aktivität nützlich ist, umfassend eine therapeutisch wirksame Menge des Peptids nach Anspruch 8 oder 9 und eine therapeutisch wirksame Menge eines reifen TGF-β in einem pharmazeutisch annehmbaren Träger.

12. Verfahren zum Diagnostizieren des Vorliegens einer Krankheit oder Störung, die durch die Gegenwart von reifem TGF-β in einer Serumprobe eines Patienten nachweisbar ist, umfassend die Zugabe einer markierten Form des Peptids nach Anspruch 8 oder 9 zur Serumprobe und das Testen auf die Gegenwart von markierten Komplexen aus Peptid und reifem TGF-β in der Serumprobe.

13. Peptid nach Anspruch 8 oder 9 zur Verwendung in einem Verfahren zur medizinischen Behandlung oder Diagnose.

14. Verwendung des Peptids nach Anspruch 8 oder 9 zur Herstellung eines Medikaments zum Entgegenwirken gegen eine TGF-β-Aktivität in einem Säugetier.

15. Verwendung des Peptids nach Anspruch 8 oder 9 und TGF-β zur Herstellung eines Medikaments zur Behandlung eines Säugetiers, das einer TGF-β-Behandlung bedarf.

## Patentansprüche (Patentansprüche für folgende(n) Vertragsstaat(en): ES)

1. Verfahren, umfassend die Herstellung einer isolierten Nukleinsäure-Sequenz, die eine für das in Fig. 8 dargestellte Peptid kodierende DNA-Sequenz oder Analoge oder Varianten davon umfaßt und die biologische Aktivität eines latenzassoziierten Peptids besitzt, vorausgesetzt, daß die Sequenz nicht auch für einen reifen TGF-β kodiert.

2. Verfahren nach Anspruch 1, worin das von der Nukleinsäure-Sequenz kodierte Peptid eine zumindest 80%-ige Aminosäuresequenz-Identität mit dem rekombinanten, latenzassoziierten Peptid aus Fig. 8 aufweist.

3. Verfahren, umfassend die Herstellung eines Expressionsvektors, der die Nukleinsäure-Sequenz nach Anspruch 1 oder 2 umfaßt, die mit Steuersequenzen operabel verbunden ist, die von einem mit dem Vektor transformierten Wirt erkannt werden.

4. Verfahren nach Anspruch 3, worin der Vektor der in Fig. 9 (Forts. a) dargestellte Vektor pSV16B-LAP-SER ist.

5. Verfahren nach Anspruch 3, worin der Vektor der unter ATCC 68.150 hinterlegte Vektor pSV16B-LAP-ARG ist.

6. Mit dem Expressionsvektor nach irgendeinem der Ansprüche 3 - 5 transformierte Wirtszelle.

7. Verfahren zur Herstellung eines latenzassoziierten Peptids, umfassend das Kultivieren der Zellen nach Anspruch 6 zum Exprimieren von Nukleinsäure, die für das Peptid in der Wirtszellenkultur kodiert.

8. Verfahren, umfassend die Herstellung eines latenzassoziierten Peptids mit einem Molekulargewicht von etwa 75.000, gemessen durch nicht-reduzierende SDS-PAGE, das in der Lage ist, einer biologischen Aktivität von reifem TGF-β entgegenzuwirken, undvollständig frei von Ursprungsproteinen ist.

9. Verfahren zur Herstellung einer pharmazeutischen Zusammensetzung, die zum Entgegenwirken gegen eine TGF-β-Aktivität dient, umfassend das Vermischen einer therapeutisch wirksamen Menge des Peptids nach Anspruch 8 mit einem pharmazeutisch annehmbaren Träger.

10. Verfahren zur Herstellung einer pharmazeutischen Zusammensetzung, die aufgrund einer TGF-β-Aktivität nützlich ist, umfassend das Vermischen einer therapeutisch wirksamen Menge des Peptids nach Anspruch 8 mit einem pharmazeutisch annehmbaren Träger.

11. Verfahren zum Diagnostizieren des Vorliegens einer Krankheit oder Störung, die durchdie Gegenwart von reifem TGF-β in einer Serumprobe eines Patienten nachweisbar ist, umfassend die Zugabe einer markierten Form des Peptids nach Anspruch 8 zur Serumprobe und das Testen auf die Gegenwart von markierten Komplexen aus Peptid und reifem TGF-β in der Serumprobe.

12. Verwendung des Peptids nach Anspruch 8 zur Herstellung eines Medikaments zum Entgegenwirken gegen eine TGF-β-Aktivität in einem Säugetier.

13. Verwendung des Peptids nach Anspruch 8 und TGF-β zur Herstellung eines Medikaments zur Behandlung eines Säugetiers, das einer TGF-β-Behandlung bedarf.

## Patentansprüche (Patentansprüche für folgende(n) Vertragsstaat(en): GR)

1. Isolierte Nukleinsäure-Sequenz, umfassend eine für das in Fig. 8 dargestellte Peptid kodierende DNA-Sequenz oder Analoge oder Varianten davon, die die biologische Aktivität eines latenzassoziierten Peptids besitzt, vorausgesetzt, daß die Sequenz nicht auch für einen reifen TGF-β kodiert.

2. Nukleinsäure-Sequenz nach Anspruch 1, worin das von der Nukleinsäure-Sequenz kodierte Peptid eine zumindest 80%-ige Aminosäuresequenz-Identität mit dem rekombinanten, latenzassoziierten Peptid aus Fig. 8 aufweist.

3. Expressionsvektor, umfassend die Nukleinsäure-Sequenz nach Anspruch 1 oder 2, die mit Steuersequenzen operabel verbunden ist, die von einem mit dem Vektor transformierten Wirt erkannt werden.

4. Vektor nach Anspruch 3, der der in Fig. 9 (Forts. a) dargestellte Vektor pSV16B-LAP-SER ist.

5. Vektor nach Anspruch 3, der der unter ATCC 68.150 hinterlegte Vektor pSV16B-LAP-ARG ist.

6. Mit dem Expressionsvektor nach irgendeinem der Ansprüche 3 - 5 transformierte Wirtszelle.

7. Verfahren zur Herstellung eines latenzassoziierten Peptids, umfassend das Kultivieren der Zellen nach Anspruch 6 zum Exprimieren von Nukleinsäure, die für das Peptid in der Wirtszellenkultur kodiert.

8. Latenzassoziiertes Peptid, das nach dem Verfahren nach Anspruch 7 erhalten werden kann.

9. Latenzassoziiertes Peptid mit einem Molekulargewicht von etwa 75.000, gemessen durch nicht-reduzierende SDS-PAGE, das in der Lage ist, einer biologischen Aktivität von reifem TGF-β entgegenzuwirken, undvollständig frei von Ursprungsproteinen ist.

10. Verfahren zur Herstellung einer pharmazeutischen Zusammensetzung, die zum Entgegenwirken gegen eine TGF-β-Aktivität dient, umfassend das Vermischen einer therapeutisch wirksamen Menge des Peptids nach Anspruch 8 oder 9 mit einem pharmazeutisch annehmbaren Träger.

11. Verfahren zur Herstellung einer pharmazeutischen Zusammensetzung, die aufgrund einer TGF-β-Aktivität nützlich ist, umfassend das Vermischen einer therapeutisch wirksamen Menge des Peptids nach Anspruch 8 oder 9 mit einem pharmazeutisch annehmbaren Träger.

12. Verfahren zum Diagnostizieren des Vorliegens einer Krankheit oder Störung, die durch die Gegenwart von reifem TGF-β in einer Serumprobe eines Patienten nachweisbar ist, umfassend die Zugabe einer markierten Form des Peptids nach Anspruch 8 oder 9 zur Serumprobe und das Testen auf die Gegenwart von markierten Komplexen aus Peptid und reifem TGF-β in der Serumprobe.

13. Verwendung des Peptids nach Anspruch 8 oder 9 zur Herstellung eines Medikaments zum Entgegenwirken gegen eine TGF-β-Aktivität in einem Säugetier.

14. Verwendung des Peptids nach Anspruch 8 oder 9 und TGF-β zur Herstellung eines Medikaments zur Behandlung eines Säugetiers, das einer TGF-β-Behandlung bedarf.

## Revendications (Revendications pour l'(les) Etat(s) contractant(s) suivant(s): AT, BE, CH, DE, DK, FR, GB, IT, LI, LU, NL, SE)

1. Séquence d'acide nucléique isolée comprenant une séquence d'ADN codant pour le peptide présenté à la Figure 8, ou des analogues ou des variants de celui-ci, doués de l'activité biologique d'un peptide associé à une latence, à la condition que cette séquence ne code pas pour un TGF-β mature.

2. Séquence d'acide nucléique selon la revendication 1 dans laquelle le peptide encodé par la séquence d'acide nucléique présente au moins 80% d'homologie de séquence d'acides aminés avec le peptide recombinant associé à une latence de la Figure 8.

3. Vecteur d'expression comprenant la séquence d'acide nucléique selon la revendication 1 ou la revendication 2 liée de manière fonctionnelle aux séquences de régulation reconnues par un hôte transformé par le vecteur.

4. Vecteur selon la revendication 3 qui est le pSV16B-LAP-SER tel qu'indiqué à la Figure 9 ( et la suite a).

5. Vecteur selon la revendication 3 qui est le pSV16B-LAP-ARG déposé sous la référence ATCC 68150.

6. Cellule hôte transformée par le vecteur d'expression selon l'une quelconque des revendications 3 à 5.

7. Procédé de production d'un peptide associé à une latence, lequel procédé comprend la mise en culture de cellules selon la revendication 6 en vue d'exprimer l'acide nucléique codant pour le peptide dans la culture de cellules hôtes.

8. Peptide associé à une latence comme celui pouvant être obtenu par le procédé selon la revendication 7.

9. Peptide associé à une latence ayant un poids moléculaire d'environ 75 000 tel que mesuré par PAGE-SDS en milieu non réducteur et capable d'antagoniser une activité biologique du TGF-β mature, lequel peptide est totalement exempt de protéines de source.

10. Composition pharmaceutique utile pour antagoniser une activité du TGF-β comprenant une quantité thérapeutiquement efficace du peptide selon la revendication 8 ou la revendication 9 en association avec un véhicule pharmaceutiquement acceptable.

11. Composition pharmaceutique utile car douée d'une activité TGF-β comprenant une quantité thérapeutiquement efficace du peptide selon la revendication 8 ou la revendication 9 et une quantité thérapeutiquement efficace d'un TGF-β mature en association avec un véhicule pharmaceutiquement acceptable.

12. Procédé pour diagnostiquer une maladie ou un trouble pouvant être décelé par la présence de TGF-β mature dans un échantillon sérique d'un malade comprenant l'adjonction de peptide selon la revendication 8 ou la revendication 9 à l'état marqué à un échantillon de sérum et le dosage pour détecter la présence de complexes marqués du peptide et du TGF-β mature dans l'échantillon de sérum.

13. Peptide selon la revendication 8 ou la revendication 9 destiné à être utilisé dans un procédé de traitement médical ou diagnostique.

14. Utilisation du peptide selon la revendication 8 ou la revendication 9 dans la préparation d'un médicament destiné à antagoniser une activité du TGF-β chez un mammifère.

15. Utilisation du peptide selon la revendication 8 ou la revendication 9 et de TGF-β dans la préparation d'un médicament destiné au traitement d'un mammifère nécessitant un traitement par le TGF-β.

## Revendications (Revendications pour l'(les) Etat(s) contractant(s) suivant(s): ES)

1. Procédé qui comprend la production d'une séquence d'acide nucléique isolée comprenant une séquence d'ADN codant pour le peptide présenté à la Figure 8, ou des analogues ou des variants de celui-ci, doués de l'activité biologique d'un peptide associé à une latence, à la condition que cette séquence ne code pas pour un TGF-β mature.

2. Procédé selon la revendication 1 dans lequel le peptide encodé par la séquence d'acide nucléique présente au moins 80% d'homologie de séquence d'acides aminés avec le peptide recombinant associé à une latence de la Figure 8.

3. Procédé qui comprend la production d'un vecteur d'expression comprenant la séquence d'acide nucléique selon la revendication 1 ou la revendication 2 liée de manière fonctionnelle aux séquences de régulation reconnues par un hôte transformé par le vecteur.

4. Procédé selon la revendication 3 dans lequel le vecteur est le pSV16B-LAP-SER tel qu'indiqué à la Figure 9 ( et la suite a).

5. Procédé selon la revendication 3 dans lequel le vecteur est le pSV16B-LAP-ARG déposé sous la référence ATCC 68150.

6. Cellule hôte transformée par le vecteur d'expression selon l'une quelconque des revendications 3 à 5.

7. Procédé de production d'un peptide associé à une latence, lequel procédé comprend la mise en culture de cellules selon la revendication 6 en vue d'exprimer l'acide nucléique codant pour le peptide dans la culture de cellules hôtes.

8. Procédé qui comprend la production d'un peptide associé à une latence ayant un poids moléculaire d'environ 75 000 tel que mesuré par PAGE-SDS en milieu non réducteur et capable d'antagoniser une activité biologique du TGF-β mature, lequel peptide est totalement exempt de protéines de source.

9. Procédé de préparation d'une composition pharmaceutique utile pour antagoniser une activité du TGF-β, lequel procédé comprend le mélange d'une quantité thérapeutiquement efficace du peptide selon la revendication 8 avec un véhicule pharmaceutiquement acceptable.

10. Procédé de préparation d'une composition pharmaceutique utile car douée d'une activité du TGF-β, lequel procédé comprend le mélange d'une quantité thérapeutiquement efficace du peptide selon la revendication 8 avec un véhicule pharmaceutiquement acceptable.

11. Procédé pour diagnostiquer une maladie ou un trouble pouvant être décelé par la présence de TGF-β mature dans un échantillon sérique d'un malade comprenant l'adjonction de peptide selon la revendication 8 à l'état marqué à un échantillon de sérum et le dosage pour détecter la présence de complexes marqués du peptide et du TGF-β mature dans l'échantillon de sérum.

12. Utilisation du peptide selon la revendication 8 dans la préparation d'un médicament destiné à antagoniser une activité du TGF-β chez un mammifère.

13. Utilisation du peptide selon la revendication 8 et de TGF-β dans la préparation d'un médicament destiné au traitement d'un mammifère nécessitant un traitement par le TGF-β.

## Revendications (Revendications pour l'(les) Etat(s) contractant(s) suivant(s): GR)

1. Séquence d'acide nucléique isolée comprenant une séquence d'ADN codant pour le peptide présenté à la Figure 8, ou des analogues ou des variants de celui-ci, doués de l'activité biologique d'un peptide associé à une latence, à la condition que cette séquence ne code pas pour un TGF-β mature.

2. Séquence d'acide nucléique selon la revendication 1 dans laquelle le peptide encodé par la séquence d'acide nucléique présente au moins 80% d'homologie de séquence d'acides aminés avec le peptide recombinant associé à une latence de la Figure 8.

3. Vecteur d'expression comprenant la séquence d'acide nucléique selon la revendication 1 ou la revendication 2 liée de manière fonctionnelle aux séquences de régulation reconnues par un hôte transformé par le vecteur.

4. Vecteur selon la revendication 3 qui est le pSV16B-LAP-SER tel qu'indiqué à la Figure 9 ( et la suite a).

5. Vecteur selon la revendication 3 qui est le pSV16B-LAP-ARG déposé sous la référence ATCC 68150.

6. Cellule hôte transformée par le vecteur d'expression selon l'une quelconque des revendications 3 à 5.

7. Procédé de production d'un peptide associé à une latence, lequel procédé comprend la mise en culture de cellules selon la revendication 6 en vue d'exprimer l'acide nucléique codant pour le peptide dans la culture de cellules hôtes.

8. Peptide associé à une latence comme celui pouvant être obtenu par le procédé selon la revendication 7.

9. Peptide associé à une latence ayant un poids moléculaire d'environ 75 000 tel que mesuré par PAGE-SDS en milieu non réducteur et capable d'antagoniser une activité biologique du TGF-β mature, lequel peptide est totalement exempt de protéines de source.

10. Procédé de préparation d'une composition pharmaceutique utile pour antagoniser une activité du TGF-β, lequel procédé comprend le mélange d'une quantité thérapeutiquement efficace du peptide selon la revendication 8 ou la revendication 9 avec un véhicule pharmaceutiquement acceptable.

11. Procédé de préparation d'une composition pharmaceutique utile car douée d'une activité du TGF-β, lequel procédé comprend le mélange d'une quantité thérapeutiquement efficace du peptide selon la revendication 8 ou la revendication 9 avec un véhicule pharmaceutiquement acceptable.

12. Procédé pour diagnostiquer une maladie ou un trouble pouvant être décelé par la présence de TGF-β mature dans un échantillon sérique d'un malade comprenant l'adjonction de peptide selon la revendication 8 ou la revendication 9 à l'état marqué à un échantillon de sérum et le dosage pour détecter la présence de complexes marqués du peptide et du TGF-β mature dans l'échantillon de sérum.

13. Utilisation du peptide selon la revendication 8 ou la revendicaiton 9 dans la préparation d'un médicament destiné à antagoniser une activité du TGF-β chez un mammifère.

14. Utilisation du peptide selon la revendication 8 ou la revendication 9 et de TGF-β dans la préparation d'un médicament destiné au traitement d'un mammifère nécessitant un traitement par le TGF-β.
